# EUROPEAN PATENT SPECIFICATION

(11) **EP 2 565 281 B1**
(45) Date of publication and mention of the grant of the patent: **20.07.2016**
(21) Application number: 12182195.3
(22) Date of filing: 29.08.2012
(51) Int. Cl.: C12Q 1/68

(54) **Dye blends for qPCR and High Resolution Melting**
Farbstoffmischungen für qPCR und High Resolution Melting
Mélanges de colorants pour la qPCR et le High Resolution Melting

(30) Priority: 29.08.2011 US 201161528315 P
(43) Date of publication of application: 06.03.2013
(73) Proprietor: Thermo Fisher Scientific Inc., Waltham MA 02454 (US)
(72) Inventor: Kavanagh, Ian, 6006 Luzern (CH); Leake, Devin, Denver, CO Colorado 80238 (US); Ball, Glyn, Staines, Middlesex TW18 2LQ (GB)
(74) Representative: Instone, Alicia Claire

(56) References cited:
- EP-A1- 2 226 395
- WO-A1-2010/071602
- WO-A2-2004/038038
- WO-A2-2009/046010
- CN-A- 101 724 295
- ANNE C EISCHEID: "SYTO dyes and EvaGreen outperform SYBR Green in real-time PCR", BMC RESEARCH NOTES, BIOMED CENTRAL LTD, GB, vol. 4, no. 1, 28 July 2011 (2011-07-28), page 263, XP021106169, ISSN: 1756-0500, DOI: 10.1186/1756-0500-4-263

### SEQUENCE LISTING

<110> Thermo Fisher Scientific Inc.
<120> DYE BLENDS
<130> EPP7003
<150> 61/528,315
   <151> 2011-08-29
<160> 10
<170> PatentIn version 3.5
<210> 1
   <211> 20
   <212> DNA
   <213> Artificial Sequence
<220>
   <223> Synthetic Construct - GAPDH
<400> 1
   acagtcagcc gcatcttctt 20
<210> 2
   <211> 20
   <212> DNA
   <213> Artificial Sequence
<220>
   <223> Synthetic Construct - GAPDH
<400> 2
   acgaccaaat ccgttgactc 20
<210> 3
   <211> 210
   <212> DNA
   <213> Artificial Sequence
<220>
   <223> Synthetic Construct - 210 bp region
<400> 3
   ggatggaaga gatgacaagg acataggaga taaatatcag gcctatgggc acaaaaatca        60
   caaatgaact tacaccataa tttattatct cattgatagt ggtatcaatg caagaaagtt       120
   tcatgactgg gtaaatgtca caaaagaagt ggtctaccac tgtgccacag aacggcaaat       180
   tgaacatggc tgtcacatgg agaactgcca                                        210
<210> 4
   <211> 27
   <212> DNA
   <213> Artificial Sequence
<220>
   <223> Synthetic Construct - forward primer
<400> 4
   ggatggaaga gatgacaagg acatagg 27
<210> 5
   <211> 22
   <212> DNA
   <213> Artificial Sequence
<220>
   <223> Synthetic Construct - reverse primer
<400> 5
   tggcagttct ccatgtgaca gc 22
<210> 6
   <211> 21
   <212> DNA
   <213> Artificial Sequence
<220>
   <223> Synthetic Construct - SNP A rs4656837
<400> 6
   tcaatttgcc attctgtggc a 21
<210> 7
   <211> 21
   <212> DNA
   <213> Artificial Sequence
<220>
   <223> Synthetic Construct - SNP B; SNP A/B (rs4656837)
<400> 7
   tcaatttgcc gttctgtggc a 21
<210> 8
   <211> 108
   <212> DNA
   <213> Artificial Region
<220>
   <223> Synthetic Construct - 108 bp region
<400> 8
   actgggtaaa tgtcacaaaa gaagtggtct accactgtgc cacagaacgg caaattgaac 60
   atggctgtca catggagaac tgccatagtc agaccaatgc caaaggac 108
<210> 9
   <211> 23
   <212> DNA
   <213> Artificial Sequence
<220>
   <223> Synthetic Construct - forward primer
<400> 9
   gtcctttggc attggtctga cta 23
<210> 10
   <211> 26
   <212> DNA
   <213> Artificial Sequence
<220>
   <223> Synthetic Construct - reverse primer
<400> 10
   actgggtaaa tgtcacaaaa gaagtg 26

## Description

At least two biological dyes, termed a dye blend, that bound preferentially and non-specifically to double stranded DNA (dsDNA), exhibited higher fluorescence and lower inhibition of DNA polymerase than that exhibited by each dye alone, even when each dye alone was used at a higher concentration than the concentration of that dye in the dye blend. The dye blend increased the maximum fluorescence signal at the PCR endpoint, decreased the quantification cycle (Cq), and did so without compromising polymerase catalytic activity. That is, the dye blend enhanced PCR signal detection, improved PCR detection threshold, and reduced PCR inhibition. By overcoming single dye inhibition of DNA polymerase, the dye blend saturated dsDNA to a higher percentage, which is important for HRM analysis.

The dyes bound non-specifically to DNA in that they did not require a specific DNA sequence to bind to. The dyes bound preferentially to dsDNA over single stranded DNA (ssDNA). The dye blends were evaluated at various concentrations and under various conditions to determine performance in a quantitative polymerase chain reaction assay (qPCR, also referred to as real-time PCR (rt-PCR) or real-time quantitative PCR (rt-qPCR), and a high resolution melt (HRM) analysis, also referred to as a saturation binding profile.

The dye blend, in addition to utility in qPCR and/or HRM assays, was applicable in other assays using fluorescent DNA binding dyes, including but not limited to gel electrophoresis, DNA quantification, cell staining, etc.

CN101724295A (UNIV CHINA AGRICULTURAL) describes LE Green mixed dye and applications thereof in double-stranded nucleic acid detection. The nucleic acid dye is prepared by comprising LC Green, Eva Green and proper diluent. The double-stranded nucleic acid template (genome DNA, plasmid DNA and the like) and a fluorescent dye LE Green Mix with proper concentration are mixed, and fluorescent detection instruments, such as a Modulus spectrophotometer, a fluorescent microplate reader, a real-time quantitive PCR instrument and the like, are used for quantitively detecting the fluorescence intensity generated after the dye and the purified double-stranded nucleic acid.

EP2226395A1 (UNIV PARMA[IT]) describes a method and a kit allowing to detect the presence, in analysed samples, of material obtained from two or more allergenic species comprised in the group: almond (Prunus dulcis), hazelnut (Corylus avellana), cashew (Anacardium occidentalis), peanut ( Arachis hypogea), walnut (Juglans regia), and sesame (Sesamum indicum).

### BRIEF DESCRIPTION OF THE DRAWINGS

The patent or application file contains at least one drawing executed in color. Copies of this patent or patent application publication with color drawing(s) will be provided by the Office upon request and payment of the necessary fee.
FIG. 1 shows amplification curves (FIG. 1 A) and melting peaks (FIG. 1 B) using double stranded DNA (dsDNA) binding dyes separately and in combination.
FIGS. 2A-2D show selected amplification curves from FIG. 1A.
FIG. 3 is a plot of mean quantitation cycle threshold (Cq) values (FIG. 3A) and relative fluorescence units (RFU) (FIG. 3B) from FIG. 1A.
FIG. 4 shows amplification curves (FIG. 4A) and melting peaks (FIG. 4B) using double stranded DNA (dsDNA) binding dyes separately and in combination.
FIGS. 5A-5D show selected amplification curves from FIG. 4A.
FIG. 6 is a plot of mean quantitation cycle threshold (Cq) values (FIG. 6A) and relative fluorescence units (RFU) (FIG. 6B) from FIG. 4A.
FIGS. 7A-7D show saturation curves using double stranded DNA (dsDNA) binding dyes separately and in combination.
FIG. 8 shows selected data from FIGS. 7A-7D represented as percent saturation.
FIGS. 9A- 9D show saturation curves using double stranded DNA (dsDNA) binding dyes separately and in combination.
FIG. 10 shows selected data from FIGS. 9A-9D represented as percent saturation.
FIG. 11 shows amplification curves using double stranded DNA (dsDNA) binding dyes separately and in combination.
FIGS. 12A-C shows performance of exemplary dye blends in high resolution melting compared to quantitative polymerase chain reaction.
FIG. 13 shows DNA sequencing results of SNP rs4656837.
FIGS. 14A-B shows high resolution melting (HRM) data using double stranded DNA (dsDNA) binding dyes separately and in combination.

According to a first aspect of the present invention there is provided a method for quantitative polymerase chain reaction (qPCR) as claimed in any of claims 1 to 9.

According to a second aspect of the present invention there is provided a method of analyzing a double-stranded deoxyribonucleic acid (dsDNA) in a high resolution melting assay as claimed in claim 10 or claim 11.

According to a third aspect of the present invention there is provided a kit comprising the components of the polymerase chain reaction assay and/or the high resolution melting assay as claimed in any of claims 1 to 11 and instructions for performing each of high resolution melting (HRM) and quantitative polymerase chain reaction (PCR) using the components of the kit as claimed in claim 12 or claim 13.

Also described herein is a polymerase chain reaction assay and/or a high resolution melting assay containing a fluorescent complex resulting from non-specific, non-covalent binding of a test nucleic acid to at least two and up to ten double stranded DNA binding dyes, the test nucleic acid being at least one of a control nucleic acid for quantitative PCR (qPCR), a nucleic acid amplified by qPCR, or a double-stranded DNA (dsDNA), where the dsDNA binding dyes have substantially similar excitation and emission spectra such that each of the at least two dsDNA binding dyes can be excited, and its resulting emissions detected, simultaneously, and where the combination of the dyes exhibits ≥ 50% saturation of the test nucleic acid. The excitation and emission spectra may be substantially similar when a single instrument configuration can excite and measure emission from the dyes in a single measurement. The single instrument configuration comprises a filter set selecting a range of excitation and emission wavelengths. In one example, the excitation is 488 nm ± 10 nm and the emission is 525 nm ± 10 nm. In one example, the excitation is 488 nm ± 2 nm and the emission is 525 nm ± 20 nm. In one embodiment, the excitation is 488 nm ± 40 nm and the emission is 525 nm ± 40 nm. In one embodiment, the assay uses two dyes. The dyes may be ethidium bromide, SYBR® Green, LCGREEN®, SYTO® 9, EVAGREEN®, RESOLIGHT®, Chromofy, BOXTO, monomethine dyes, and/or Canon Life Science dyes.

Also described herein is a method for quantitating and/or detecting a target nucleic acid in a biological sample. This method comprises performing a quantitative polymerase chain reaction (qPCR) on a target nucleic acid to generate an amplified target nucleic acid in the presence of at least two and up to 10 dsDNA binding dyes, exposing the biological sample containing the amplified target nucleic acid to light at a wavelength absorbed by each compound; and detecting a fluorescent emission intensity from the compounds where the intensity is proportional to the quantity of the amplified target nucleic acid in the sample.

Also described herein is a method for quantitating and/or detecting a target nucleic acid in a biological sample. This method comprises performing a quantitative polymerase chain reaction (qPCR) on a target nucleic acid to generate an amplified target nucleic acid in the presence of at least two and up to ten dsDNA binding dyes, exposing the biological sample containing the amplified target nucleic acid to light at a wavelength absorbed by each compound; and detecting a fluorescent emission intensity from the compounds where the intensity is proportional to the quantity of the amplified target nucleic acid in the sample, and where the at least two dsDNA binding dyes have substantially similar excitation and emission spectra such that the at least two dsDNA binding dyes can be excited, and their resulting emissions detected, simultaneously, where the dyes result in ≥ 50% saturation of the test nucleic acid. The excitation and emission properties of the dyes may be substantially similar when a single instrument configuration is able to excite and measure emission from the at least two dsDNA binding dyes in a single measurement. The single instrument configuration comprises a filter set which selects a range of excitation and emission wavelengths. In one example, the excitation is 488 nm ± 10 nm and the emission is 525 nm + 10nm. In one example, the excitation is 488 nm ± 2 nm and the emission is 525 nm ± 20 nm. In one embodiment, the excitation is 488 nm ± 40 nm and the emission is 525 nm ± 40 nm. Also described herein, the method uses two dyes. The dyes may be ethidium bromide; SYBR® Green, LCGREEN®, SYTO® 9, EVAGREEN®, RESOLIGHT®, Chromofy, BOXTO, monomethine dyes, and/or Canon Life Science dyes. Also described herein, the target nucleic acid is a ribonucleic acid (RNA), and the method reverse transcribes the RNA into DNA prior to performing qPCR.

Also described herein is a method of analyzing a target double-stranded deoxyribonucleic acid (dsDNA). The method comprises heating the dsDNA target to a melting temperature in the presence of at least two and up to ten dsDNA binding dyes, plotting the melting temperatures against fluorescence emitted from the dyes to generate a melting curve, and identifying a genotype of the target dsDNA from the melting curve by melting temperature, melting curve slope, and/or melting curve shape. In one embodiment, the melting curve of the target dsDNA is compared with a melting curve of a reference dsDNA to determine a genotype of the target dsDNA. The excitation and emission properties of the dyes may be substantially similar when a single instrument configuration is able to excite and measure emission from the at least two dsDNA binding dyes in a single measurement. The single instrument configuration comprises a filter set which selects a range of excitation and emission wavelengths. In one embodiment, the excitation is 488 nm ± 10 nm and the emission is 525 nm ± 10 nm. In one example, the excitation is 488 nm ± 2 nm and the emission is 525 nm ± 20 nm. In one example, the excitation is 488 nm ± 40 nm and the emission is 525 nm ± 40 nm. In one embodiment, the method uses two dyes. The dyes may be ethidium bromide; SYBR® Green, LCGREEN®, SYTO® 9, EVAGREEN®, RESOLIGHT®, Chromofy, BOXTO, monomethine dyes, and/or Canon Life Science dyes.

Also described herein is a method of detecting mutations in a target nucleic acid by performing a quantitative polymerase chain reaction (qPCR) in the presence of at least two and up to ten double-stranded DNA binding dyes to generate an amplicon, heating the amplicon to generate a melting curve, and analyzing melting temperature, melting curve slope, and/or melting curve shape to detect a mutation in a target sample.

Also described herein is a method of analyzing a target nucleic acid by (a) first performing quantitative polymerase chain reaction (qPCR) on a target nucleic acid in a biological sample in the presence of a reaction mixture comprising at least two and up to ten dsDNA binding dyes resulting in an amplified target nucleic acid, (b) exposing the sample containing the amplified target nucleic acid to light at a wavelength absorbed by each of the dyes, and (c) detecting a fluorescent emission intensity from the dyes where the intensity is proportional to the quantity of the amplified target nucleic acid in the sample; and (d) subsequently determining a genotype of the target nucleic acid in the sample by heating the amplified target to generate a melting curve, and analyzing the melting curve to determine the genotype of the target nucleic acid in the absence of additional components to the reaction mixture.

Also described herein is a method for nucleic acid analysis by combining a target nucleic acid and at least two and up to ten double stranded DNA binding dyes and an unlabeled probe configured to hybridize to a portion of the target nucleic acid to result in a mixture, incubating the mixture under conditions to hybridize the unlabeled probe to the target nucleic acid to form a probe/target duplex, measuring fluorescence from the dyes as the mixture is heated, generating a melting curve for the probe/target duplex, and analyzing the shape of the melting curve including generating a derivative curve and analyzing the shape and location of one or more melting peaks on the derivative melting curve. In one embodiment, the target nucleic acid contains at least one point mutation and the method detects the point mutation.

Also described herein is a kit comprising at least two and up to ten double stranded DNA binding dyes. The kit contains instructions for performing each of high resolution melting of a target nucleic and quantitative polymerase chain reaction using components of the kit. The kit may contain a reference nucleic acid encoding a *CFTR, BRCA1, BRCA2, MFN2, HTT, SMPD1,* or *NOD2* gene. Both qPCR and HRM may be performed using the kit.

Also described herein is a method of saturating double stranded DNA (dsDNA) with at least two and up to ten double stranded DNA binding dyes at concentrations not inhibitory to quantitative polymerase chain reaction (qPCR). The method comprises providing the dyes to a dsDNA at a concentration that provides at least 90% maximal fluorescent signal, where the concentration of the dyes does not substantially inhibit qPCR of the dsDNA, and where the blend of dsDNA-binding fluorescent dyes is selected from at least two and up to ten of ethidium bromide; SYBR® Green, LCGREEN®, SYTO® 9, EVAGREEN®, RESOLIGHT®, Chromofy, BOXTO, monomethine dyes, and Canon Life Science dyes.

Also described herein is a polymerase chain reaction assay and/or a high resolution melting assay containing a fluorescent complex resulting from non-specific, non-covalent binding of a test nucleic acid to at least two and up to ten double stranded DNA binding dyes selected from ethidium bromide, SYBR® Green, LCGREEN®, SYTO® 9, EVAGREEN®, RESOLIGHT®, Chromofy, BOXTO, monomethine dyes, and/or Canon Life Science dyes, where the test nucleic acid is at least one of a control nucleic acid for quantitative PCR (qPCR), a nucleic acid amplified by qPCR, or a double-stranded DNA (dsDNA), and where the dyes have substantially similar excitation and emission spectra such that each dye is excited, and its resulting emission detected, simultaneously, and where the dyes provide ≥ 50% saturation of the test nucleic acid.

Also described herein is a method for quantitating and/or detecting a target nucleic acid in a biological sample. The method comprises performing a quantitative polymerase chain reaction (qPCR) on a target nucleic acid to generate an amplified target nucleic acid in the presence of at least two and up to ten double stranded DNA binding dyes, exposing the biological sample containing the amplified target nucleic acid to light at a wavelength absorbed by dyes; and detecting a fluorescent emission intensity from the dyes where the intensity is proportional to the quantity of the amplified target nucleic acid in the sample, and where the at dyes have substantially similar excitation and emission spectra such that the dyes are excited, and their resulting emissions detected, simultaneously, where the dyes provide ≥ 50% saturation of the test nucleic acid.

When using dsDNA binding dyes to quantitate DNA, higher concentrations of dsDNA generally result in higher fluorescence. The amplicons that result from qPCR can be analyzed by melt curve analysis, e.g., a standard melt curve or HRM assay, to distinguish their sizes or characteristics, e.g., polymorphisms. While qPCR monitors fluorescence increase, HRM assays monitor fluorescence decrease as the dsDNA separates and the compound is no longer bound. When dye concentrations are used that are less than saturating, HRM assay results are equivocal; this is because the dye released upon melting tends to simply move to regions of the DNA that remain dsDNA. Dye relocation obscures potential sequence differences in the dsDNA templates. Thus, saturating concentrations of dyes are used in both qPCR and HRM assays to increase fluorescence signal and saturate dsDNA, resulting in a more sensitive assay that detected fewer/smaller differences in dsDNA.

In qPCR, increased dye binding to dsDNA permits quantifying dsDNA by measuring the quantification cycle (Cq). Cq is the cycle at which fluorescence crosses a certain value during DNA amplification. A lower Cq indicates a greater quantity of DNA at the start of amplification. The higher fluorescence signal resulting from increased dye concentration in the amplification reaction permits DNA quantitation at a lower Cq value, e.g., the assay becomes more sensitive.

An increased concentration of dsDNA binding dye in qPCR provides benefits, but also inhibits the DNA polymerase enzyme in the reaction mixture in a concentration-dependent manner. Increasing a dye concentration beyond an optimal value quickly inhibits DNA polymerase, resulting in reduced product synthesis and, in turn, reduced yield, lower qPCR endpoint, and increased Cq, i.e., reduced assay sensitivity and efficiency.

The inventive dye blends provided increased assay sensitivity and increased efficiency to both qPCR and HRM assays.

Combinations of at least two structurally similar dyes, and combinations of at least two structurally distinct dyes were evaluated to determine if blending or mixing dyes having differing binding mechanisms, characteristics, modes led to enhanced performance in either qPCR and/or high resolution melting (HRM) assays. The dyes in the blends can have similar, complimentary, or different properties and structures. The dye blends have substantially similar excitation and emission spectra. Excitation and emission spectra are substantially similar when the dyes can be excited and detected at the same time by an instrument; a single instrument configuration can excite and measure emission from the at least two dsDNA binding dyes in a single measurement. A single instrument configuration has a filter set that selects a range of excitation and emission wavelengths. The spectra can be read in the same channel/same filter set of an instrument. In one embodiment, the channel/filter set is a SYBR Green channel/filter set. In one embodiment, the channel/filter set provides excitation at 488 nm ± 40 nm and emission at 525 nm ± 40 nm. In one embodiment, the channel/filter set provides excitation at 488 nm ± 20 nm and emission at 525 nm ± 20 nm. In one embodiment, the channel/filter set provides excitation at 488 nm ± 10 nm and emission at 525 nm ± 10 nm. In one embodiment, any blend of from two dyes and up to ten dyes, where each dye in the blend has an absorbance maximum close to 488 nm and emission maximum close to 525 nm are used. In one embodiment, two dyes are used in the dye blend. In one embodiment, more than two dyes are used in the dye blend. In one embodiment, three dyes are used in the dye blend. In one embodiment, four dyes are used in the dye blend. In one embodiment, five dyes are used in the dye blend. In one embodiment, six dyes are used in the dye blend. In one embodiment, seven dyes are used in the dye blend. In one embodiment, eight dyes are used in the dye blend. In one embodiment, nine dyes are used in the dye blend. In one embodiment, ten dyes are used in the dye blend.

Quantitative PCR applications include research (quantitative gene transcription, DNA changes over time responsive to various environments or agents, progression of cell differentiation) and clinical diagnostics (e.g., DNA present in infectious diseases, cancer, genetic abnormalities). Quantitative-PCR amplifies a target DNA or a copy DNA derived from RNA by reverse transcription, to simultaneously detect it and quantify it in real time, i.e., as amplification occurs rather than when amplification is complete. In qPCR the quantity is reported as an absolute number, or as the number of copies, or as a relative amount when normalized to either the amount of DNA at the outset of the PCR (DNA input), or the amount of DNA produced from housekeeping genes. Use of qPCR in combination with reverse transcription (RT) permits quantification of ribonucleic acid, both coding RNA (messenger RNA (mRNA)), and non-coding RNAs.

Products of qPCR are typically detected by one of two methods: (1) measuring non-specific fluorescence from dyes that intercalate with any dsDNA; or (2) measuring specific fluorescence from probes that bind to specific DNA sequences and that are labeled with a fluorescent dye (reporter) that is detectable only when the probe is hybridized with its complementary DNA target.

In method (1), the dye binds non-specifically to, and/or intercalates with, dsDNA causing the dye to fluoresce. As the dsDNA is amplified during qPCR cycles, the fluorescence increases and is determined at each amplification cycle, and the fluorescence increase is used to determine the DNA concentration in comparison to a standard dilution series of known dsDNA, or to a relative concentration in comparison to another target, such as a stably expressed reference gene. The reagents are prepared as with standard PCR, and adding a fluorescent dye. The PCR analyzer includes a fluorescence detector.

In method (2), an oligonucleotide probe is added to the qPCR reaction mixture. The oligonucleotide sequence of the probe is substantially complementary to a region of the target DNA, and the probe contains a dye whose fluorescence is detected or enhanced only if the probe is hybridized to the target DNA. As the quantity of target DNA is increased during amplification cycles, the quantity of probe that can hybridize to the target sequence is increased and hence fluorescence is increased. The increase in fluorescence is used to determine the DNA concentration in the sample by comparing it to a standard dilution series of a known DNA, or to a relative concentration in comparison to another target, such as a stably expressed reference gene. The reagents are prepared as with standard PCR, and adding a fluorescently-labeled probe. The PCR analyzer includes a fluorescence detector.

There are instances when the above detection methods are combined. One example is probes labeled with an indirectly excitable fluorophore can be used in conjunction with a fluorescent dye that intercalates into dsDNA and when the probe hybridizes to a target strand in the reaction mixture and the dsDNA binding dye is excited, the fluorophore of the probe emits (U.S. Patent No. 7,632,642). Another example is the use of a probe oligonucleotide labeled with dsDNA binding dye (U.S. Published Patent Application No. 2008/0220415 A1)

In either method, relative DNA concentrations during the exponential PCR phase are determined by plotting fluorescence versus PCR cycle number on a logarithmic scale; the exponentially-increasing DNA quantity results in a straight line. The threshold for detecting fluorescence above background is determined. The cycle at which the sample fluorescence exceeds this threshold is termed the quantitation cycle threshold, Cq. The DNA quantity theoretically doubles every cycle during the exponential phase, permitting calculation of the relative amount of DNA. For example, if Cq of sample A is reached three times sooner than Cq of sample B, sample A contains 2³ = 8 times more DNA template.

High resolution melt (HRM) analysis detects genetic aberrations in dsDNA. It characterizes dsDNA based on thermal dissociation behavior to determine and identify differences in sequence, length, GC content, and/or strand complementarity. HRM analysis exceeds traditional DNA dissociation analysis, also termed DNA melting curve analysis, in the quality and quantity of the resulting information. HRM analysis detects genetic aberrations such as mutations, including single base changes (single nucleotide polymorphisms or SNPs), polymorphisms, epigenetic differences, etc. It thus is used for mutation discovery (gene scanning), screening for loss of heterozygosity, DNA fingerprinting, SNP genotyping, characterization of haplotype blocks, DNA methylation analysis, DNA mapping, species identification, somatic acquired mutation ratios, HLA compatibility typing, allelic prevalence in a population, and identification of candidate predisposition genes. HRM is more cost effective than DNA sequencing, is applicable to large scale rapid and accurate genotyping, and is a relatively simple technique.

In one embodiment, HRM analysis is performed after amplifying a DNA region of interest (e.g., one containing a mutation) after qPCR. This amplified region, amplicon, is heated from about 50°C to about 95°C and, at a point during this heating process, the amplicon melting temperature is reached and the two strands of DNA separate ("melt" apart). The melting temperature, Tₘ, is the temperature at which one-half of the DNA strands are double-stranded and one-half are single-stranded. Tₘ depends on both the DNA length and its specific nucleotide sequence composition. The precise temperature, determined to a fraction of a degree, at which melting occurs is monitored using a fluorescent dye that binds and/or intercalates specifically to only dsDNA; dye bound to dsDNA exhibits intense fluorescence, while dye in the presence of single stranded DNA (ssDNA) or "melted" DNA exhibits minimal fluorescence. Thus, at the outset of HRM analysis, the sample exhibits high fluorescence due to numerous amplicons. Upon heating, the fluorescence in the sample decreases due to DNA melting and dye un-binding.

A fluorometer measures emitted fluorescence, which is plotted against temperature to generate a melting curve. The temperature at which dsDNA separates ("melts") depends upon its base sequence. For example, two samples with the same amplicon yield identical melting curves because each amplicon has the identical sequence. However, if an amplicon contains a sequence with a mutation, the temperature at which the dsDNA separates ("melts") differs, yielding a different melting curve from the sample containing a non-mutated amplicon.

In one embodiment, HRM analysis is performed on genomic DNA to determine if a mutation is present. Genomic DNA contains two alleles, leading to three mutation outcomes: (1) neither allele contains a mutation (wild type), (2) one allele contains a mutation (heterozygote), (3) both alleles contain a mutation (homozygote). HRM analysis of genomic DNA under each mutation outcome yields a different melting curve. Thus, when analyzing genomic DNA, a mutation in a gene associated with, e.g., breast cancer, can be screened for and the patient can be assessed as having a wild type, homozygous, or heterozygous genotype. Examples of genetic mutations correlated with a disease, and detectable with HRM analysis, include but are not limited to mutations in cystic fibrosis transmembrane conductance regulator (CFTR), BRCA1/2 (breast cancer), mitofusin-2 (MFN2; Charcot-Marie-Tooth disease), Huntingtin (HTT; Huntington's disease), sphingomyelin phosphodiesterase 1 (SMPD1; Niemann-Pick disease), NOD2 (nucleotide-binding oligomerization domain containing 2; Crohn's disease), etc.

Because dsDNA with no mis-matches has 100% base pair affinity, a first temperature is required to separate the strands, but where the dsDNA has mis-matches, and thus has less than 100% base pair affinity, a second temperature is required to separate the strands. The second temperature may be less than or greater than the first temperature, depending upon the specific DNA composition, e.g., a G →A mutation could lower the overall Tₘ. A method to detect mutations in a target nucleic acid performs qPCR in the presence of at least a dye blend to generate an amplicon, then heating the amplicon to generate a melting curve, and then analyzing the melting temperature Tₘ, melting curve slope, and/or melting curve shape to detect a mutation in a target sample. Analysis software plots the relative fluorescence unit (RFU) data collected during generation of the melting curve as a function to of temperature. Melting curves can be normalized adjusting pre-melt and post-melt regions with sliders. For better visual identification the adjusted region of the melting curve can be generated to a difference curve. Difference curve analysis shows the difference in fluorescence between a well and the fluorescence of a reference curve. The reference curve is generated from the average fluorescence of all the curves within a selected reference group.

HRM melting curves can discriminate between samples based on curve shape and/or curve shift. Curve shape analysis utilizes detailed shapes in the curve itself. Curve shift analysis utilizes thermal offset, or shift, of a curve from other curves.

Raw data may be normalized before being used in HRM curves; typical plots are fluorescence units (ordinate) versus temperature (abscissa), similar to PCR amplification plots substituting temperature (abscissa) for cycle number (abscissa). In HRM analysis plots, as with rt-PCR plots, fluorescence is normalized to a 0% to 100% scale. In one embodiment, the temperature (abscissa) is also normalized to compensate for well-to-well temperature measurement variations among samples. In one embodiment, differences in melting curve shape are analyzed by subtracting each curve from a reference curve, which results in automatic clustering the samples into groups with similar melting curves e.g., samples with heterozygote gene mutations versus samples with homozygote mutations. Information and recommendations how HRM data should be analyzed are known in the art, e.g., van der Stoep et al., 2009, Diagnostic guidelines for High-Resolution Melting Curve (HRM) Analysis: An Interlaboratory Validation of BRCA1 Mutation Scanning Using the 96-Well LightScanner™, Human Mutation 30 (6): 899-909; Montgomery et al., 2007, Simultaneous mutation scanning and genotyping by high-resolution DNA melting analysis, Nature Protocols (2) 1: 59-66; Liew et al. 2004, Genotyping of single-nucleotide polymorphisms by high-resolution melting of small amplicons, Clin Chem 50: 1156-1164; and Wittwer et al. 2003, High-resolution genotyping by amplicon melting analysis using LCGreen. Clin Chem 49: 853-860.

Commercially available instruments that contain software programs for each of these analyses are typically used, e.g., Viia 7, Stepone Plus or 7500 Real-Time PCR System (Applied Biosystems); Lightcyler 480 System (Roche); PikoReal Real-Time PCR system (Thermo Scientific); Rotorgene Q (Qiagen); CFX Real-Time Detection Systems (BioRad); Eco Real-Time PCR System (Illumina); or LightScanner System (Idaho Technology).

A hypothetical melt curve plots temperature versus fluorescence, where dsDNA binding dyes fluoresce in the presence of dsDNA but either do not fluoresce or have substantially decreased fluorescence in the presence of ssDNA. A melt curve plot typically shows the transition from dsDNA high fluorescence initial, pre-melt, phase, through the sharp fluorescence decrease of the melt phase where dsDNA is separated forming ssDNA, to base fluorescence at the final, post-melt, phase where only ssDNA is present. Fluorescence decreases as DNA intercalating dye is released from dsDNA as it separates into ssDNA. The midpoint of the melt phase, at which the rate of change in fluorescence is greatest, defines the melting temperature Tₘ of the specific DNA fragment being analyzed.

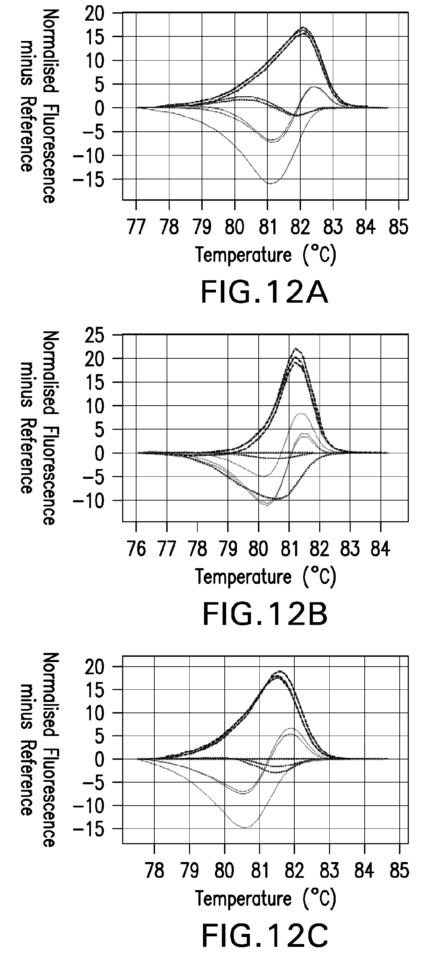

Intercalating dyes that saturate dsDNA result in homogeneous staining of homo-or heteroduplex DNA (FIGS. 12A, B, C). FIG. 12A show SNP B genotype (dark dashed line), SNP A genotype (dark dotted line), SNP AB genotype (gray solid line) (1X LC Green). FIG. 12B shows SNP B genotype (dark dashed line), SNP A genotype (dark dotted line), SNP AB genotype (gray solid line) (20 µM V11-02190). FIG. 12C shows SNP B genotype (dark dashed line), SNP A genotype (dark dotted line), SNP AB genotype (gray solid line) (0.25X LC Green and 20 µM V11-02190). Commercially available HRM analysis dyes which are suitable for the described dye blends include SYTO® 9 (Invitrogen Corp., Carlsbad CA), LCGREEN® (Idaho Technologies, Salt Lake City UT), EVAGREEN® (Biotium Inc, Hayward CA), PO-PRO™-1, BO-PRO™-1, SYTO® 43, SYTO® 44, SYTO® 45, SYTOX® Blue, POPO™-1, POPO™-3, BOBO™-1, BOBO™-3, LO-PRO™-1, JO-PRO™-1, YO-PRO®-1, TO-PRO®-1, SYTO® 11, SYTO® 13, SYTO® 15, SYTO® 16, SYTO® 20, SYTO® 23, TOTO™-3, YOYO®-3 (Molecular Probes, Inc., Eugene OR), GelStar® (Cambrex Bio Science Rockland Inc., Rockland ME), and thiazole orange (Aldrich Chemical Co., Milwaukee WI). Dyes suitable for HRM analysis have minimal inhibition and low toxicity in an amplification reaction and can thus be used at higher concentrations for greater dsDNA saturation. A dye that saturates dsDNA results in higher resolution fluorescence signals, because there is less dynamic dye redistribution to non-denatured regions of the DNA during melting. The result is greater melt sensitivity and higher resolution melt profiles.

Also described herein, the dsDNA binding dyes of the blend are selected from at least one of the following classes of dsDNA binding dyes: ethidium bromide, SYBR® Green (Life Technologies, U.S. Patent Nos. 5,436,134, 5,658,751; PCT/US94/04127 published as WO 94/24213), LCGREEN® (Idaho Technology, U.S. Patent Nos. 7,456,281, 7,387,887), SYTO 9 (Molecular Probes), EVAGREEN® (Biotium U.S. Patent No. 7,776,567), RESOLIGHT® (Roche), Chromofy (TAATA Biocenter), BOXTO (TAATA Biocenter), monomethine dyes (Dyomics, PCT/US08/78277 published as WO 2009/046010), Canon Life Science dyes (U.S. Published Patent Application No. 20100167279). Also described herein, the dsDNA binding dyes of the blend are selected from the compounds in Deligeorgivez et al. Intercalating Cyanine Dyes for Nucleic Acid Detection, Recent Patents on Material Science, 2009, 2, 1-26. Also described herein, the dsDNA binding dyes of the blend are selected from Thiazole Orange (TO), Oxazole Yellow (YO), homodimeric styrylcyanine dyes (Abbott Laboratories); PO-PRO-1 (λex/em = 435/455 nm), BO-PRO-1 (λex/em = 462/481 nm), YO-PRO-1 (λex/em = 491/509 nm), TO-PRO-1 (λex/em = 515/531 nm), SYTO, SYTOX, SYBR Gold, SYBR Green I, SYBR Green II, BEBO, BETO, BOXTO, BOXBO, PO-PRO-3 (λex/em = 539/567 nm), BO-PRO-3 (λex/em = 575/599 nm), YO-PRO-3 (λex/em = 612/631 nm), TO-PRO-3 (λex/em = 642/661 nm), TO-PRO-5 (λex/em = 745/720 nm), YOYO-1 (λex/λem = 491/509 nm), TOTO-1 (λex/λem = 514/533 nm), POPO-1 (λex/em = 434/456 nm), BOBO-1 (λex/em = 462/481 nm), POPO-3 (λex/em = 534/570 nm), BOBO-3 (λex/em = 570/602 nm), YOYO-3 (λex/em = 612/631 nm), TOTO-3 (λex/em = 642/660 nm), JO-PRO-1 (λex/em = 530/545 nm), LO-PRO-1 (λex/em = 567/580 nm), JOJO-1 (λex/em = 529/545 nm), LOLO-1 (λex/em = 565/579 nm), LDS-751 (Life Technologies Inc.); EvaGreen (Biotium); LCGreen (Idaho Technology); Thiazole Blue, Thiazole Orange and Thiazole Blue heterodimer (TOTAB), Thiazole Orange-ethidium heterodimer (TOED), and Thiazole Orange and Thiazole Indolenine heterodimer (TOTIN).

Also described herein, at least one of the dsDNA binding dyes of the blend is a compound having the formula 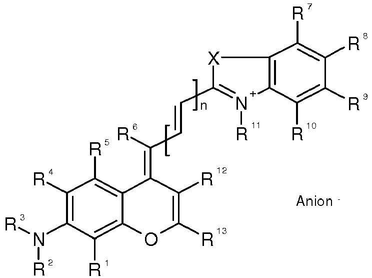 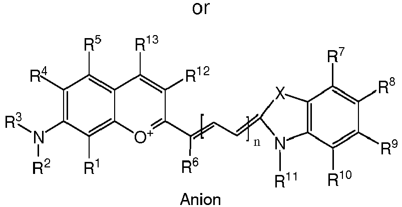
where each of R¹-R¹⁰ and R¹² is independently H or a linear or branched hydrocarbon, optionally containing one or more heteroatoms;
R¹ and R², R² and R³, R³ and R⁴, R⁴ and R⁵, R⁷ and R⁸, R⁸ and R⁹, R⁹ and R¹⁰ or R⁶ and R¹² are substituents capable of forming an aliphatic chain or ring, or an aromatic ring;
R¹¹ is a linear or branched hydrocarbon, optionally containing one ore more heteroatoms;
R¹³ is selected from
   a linear, cyclic, or branched hydrocarbon that is saturated or unsaturated, optionally containing one or more heteroatoms, optionally containing a tetraalkylammonium group;
   aryl or pyrimidyl; or
   NR¹⁴R¹⁵ where R¹⁴ and R¹⁵ are the same or different and are independently H or a hydrocarbon, optionally containing one or more heteroatoms optionally containing a tetraalkylammonium group, or R¹⁴ and R¹⁵ in combination complete a five, six, or seven membered saturated ring, optionally containing one or more heteroatoms optionally containing a quaternary ammonium group; or
   R¹³ is a linker connecting the rest of the molecule to another benzopyrylium methine dye, forming a dimer dye structure. The dyes of the dimer may be the same or different.
X is selected from the group consisting of O, S, Se, NR¹⁶ where R¹⁶ is H or a hydrocarbon optionally containing one ore more heteroatoms, and CR¹⁷R¹⁸ where R¹⁷ and R¹⁸ are the same or different and are independently a hydrocarbon optionally containing one or more heteroatoms, or in combination complete a five, six, or seven membered saturated ring, optionally containing one or more heteroatoms;
n is an integer from 0 to 3; and anion-is a counterion.

Also described herein, at least one of the dsDNA binding dyes of the blend is selected from the following compounds 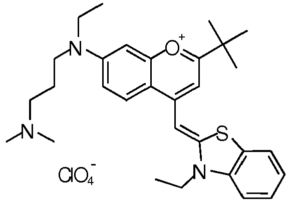 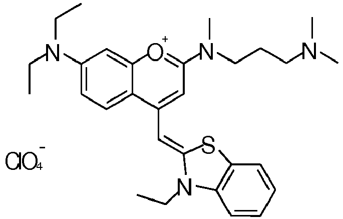 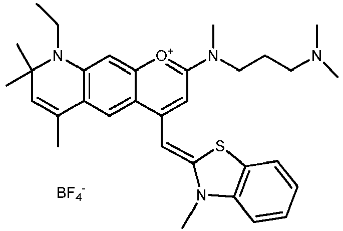 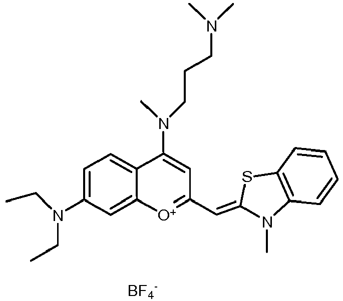 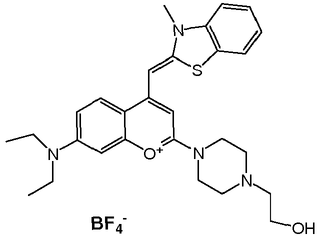 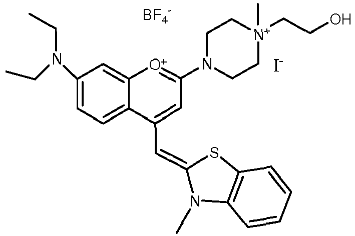 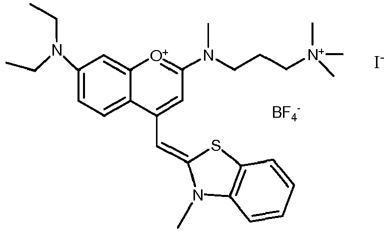 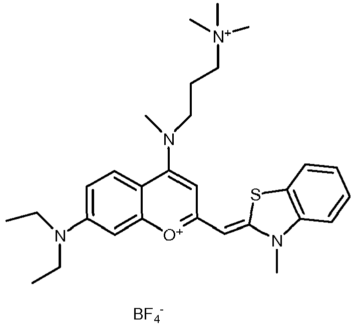 and

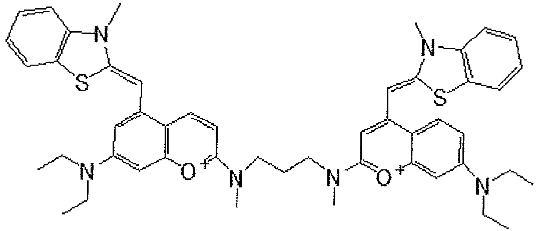

Also described herein, at least one of the dsDNA binding dyes of the blend is a compound having the formula 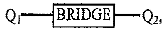 wherein BRIDGE is a substantially aliphatic, substantially neutral linker comprising from about 8 to about 150 non-hydrogen atoms; Q₁ is a dye constituent selected from a fluorescent nucleic acid dye constituent, a non-fluorescent nucleic acid dye constituent, a fluorescent non-nucleic acid dye constituent, and a non-fluorescent non-nucleic acid dye constituent; Q₂ is a dye constituent selected from a fluorescent nucleic acid dye constituent, a non-fluorescent nucleic acid dye constituent, a fluorescent non-nucleic acid dye constituent, and a non-fluorescent non-nucleic acid dye constituent; at least one dye constituent of the Q₁ dye constituent and the Q₂ dye constituent is a reporter dye constituent; at least one dye constituent of the Q₁ dye constituent and the Q₂ dye constituent is a fluorescent nucleic acid dye constituent or a non-fluorescent nucleic acid dye constituent; and the reporter dye constituent and the fluorescent nucleic acid dye constituent are optionally the same, wherein
(i) when Q₁ and/or Q₂ is a phenanthridinium dye, at least one of Q₁ and Q₂ is a phenanthridinium dye has the structure of Formula I: 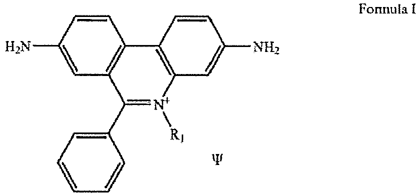 R₁ represents where BRIDGE attaches to the structure; and ψ is an anion; or
(ii) when each of Q₁ and Q₂ is an asymmetric cyanine dye, each of the Q₁ and Q₂ dye constituents has the structure of Formula II: 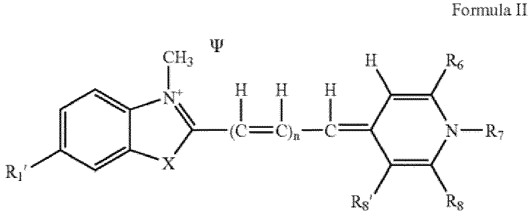
   wherein R₁' of Formula II is H; alkyl or alkenyl having 1 carbon to 6 carbons, inclusive; a halogen; -OR⁹; -SR₁₀; -NR₁₁R₁₂; -CN; -NH(C=O)R₁₃; -NHS(=O)₂R₁₄; -C(=O)NHR₁₅; or a substituent associated with minor groove binding; or represents where BRIDGE attaches to the structure;
   when R₁' of Formula II comprises at least one of R₉, R₁₀, R₁₁, R₁₂, R₁₃, R₁₄ and R₁₅, any said one of R₉, R₁₀, R₁₁, R₁₂, R₁₃, R₁₄ and R₁₅, independently, is H or alkyl having 1 carbon to 12 carbons, inclusive, optionally incorporating 1 to 2 nitrogen(s), inclusive, or an aryl;
   when R₁' of Formula II comprises R₁₁ and R₁₂, R₁₁ and R₁₂ may in combination form a 5- or 6-membered saturated or unsaturated ring, which optionally comprises at least one hetero atom selected from N and O;
   X of Formula II is selected from O and S; n of Formula II is selected from 0, 1, and 2;
   R₆ of Formula II is H; alkyl or alkenyl having 1 carbon to 10 carbons, inclusive, optionally comprising at least one hetero atom selected from N, O, and S; a halogen; -OR₁₆; -SR₁₆; -NR₁₆R₁₇; or a substituted or an unsubstituted aryl, optionally comprising 1 to 3 hetero atom(s), inclusive, selected from N, O, and S; or represents where BRIDGE attaches to the structure;
   R₇ of Formula II is H; alkyl or alkenyl having 1 carbon to 10 carbons, inclusive, optionally comprising an aryl and at least one hetero atom selected from N, O, and S; or a substituted or an unsubstituted aryl optionally comprising 1 to 3 hetero atom(s), inclusive, selected from N, O, and S; or represents where BRIDGE attaches to the structure;
   R₈ and R₈' of Formula II in combination form a fused aromatic ring, which may be further substituted 1 to 4 time(s), inclusive, independently, by C1-C2, inclusive, alkyl, C1-C2, inclusive, alkoxy, C1-C2, inclusive, alkylmercapto, or a halogen;
   each of R₁₆ and R₁₇ independently is H; alkyl having 1 carbon to 12 carbons, inclusive, optionally incorporating 1 to 2 nitrogen(s) or an aryl; or
   R₁₆ and R₁₇ may in combination form a 5- or 6-membered saturated or unsaturated ring, which optionally comprises at least one hetero atom selected from N and O;
   only one of R₁', R₆ and R₇ of Formula II represents where BRIDGE attaches to the structure; and
   ψ of Formula II is an anion; or when either Q₁ or Q₂ is an acridine dye, at least one dye constituent of the Q₁ and Q₂ dye constituents has the structure of Formula III: 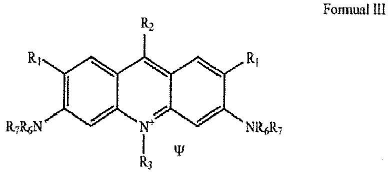
      wherein each R₁ of Formula III is independently, is H or a C1-C2, inclusive, alkyl;
      one of R₂ and R₃ of Formula III represents where BRIDGE attaches to the structure;
      when R₂ of Formula III represents where BRIDGE attaches to the structure, R₃ is H or -CH3;
      when R₃ of Formula III represents where BRIDGE attaches to the structure, R₂ is selected from H, -CH₃, -NH₂, -NHCH₃, -CN, and -C(=O)NH₂;
      each R₆ of Formula III independently, is H or a C1-C2, inclusive, alkyl;
      each R₇ of Formula III independently, is H or a C1-C2, inclusive, alkyl;
      for each pair of adjacent R₆ or R₇ and R₁, independently, R₆ or R₇ and R₁ may in combination form a 5- or 6-membered, saturated or unsaturated ring; and ψ of Formula III is an anion.

In one embodiment, at least one of the dsDNA binding dyes of the blend is a compound of the formula 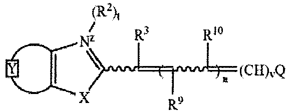
wherein the moiety Y represents an optionally-substituted fused monocyclic or polycyclic aromatic ring or an optionally-substituted fused monocyclic or polycyclic nitrogen-containing heteroaromatic ring; X is oxygen, sulfur, selenium, tellurium or a moiety selected from C(CH₃)₂ and NR¹, where R¹ is hydrogen or C₁₋₆alkyl; R² is selected from the group consisting of C₁₋₆alkyl, C₃₋₈ cycloalkyl, aryl, aryl(C₁₋₃ alkyl), hydroxyalkyl, alkoxy-alkyl, aminoalkyl, mono and dialkylaminoalkyl, trialky-lammoniumalkyl, alkylenecarboxylate, alkylenecarboxamide, alkylenesulfonate, optionally substituted cyclic heteroatom-containing moieties, and optionally substituted acyclic heteroatom-containing moieties; t=0 or 1; Z is a charge selected from 0 or 1;
R³ is selected from the group consisting of hydrogen, C₁₋₆ alkyl, and arylcarbonyl, or R² and R³ are taken together to form -CH₂)_{w}-, wherein w is 1 to 5;
R⁹ and R¹⁰ are each independently selected from the group consisting of hydrogen, C₁₋₆ alkyl, and arylcarbonyl; n=0, 1, or 2; and v=0 or 1; with the proviso that v=0 when R² and R³ are not taken together to form -(CH₂)_{w}-; wherein when v=0, Q is an heterocycle selected from the group of structures consisting of: 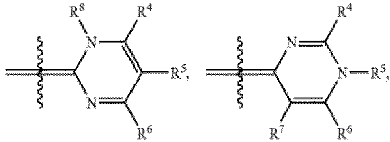 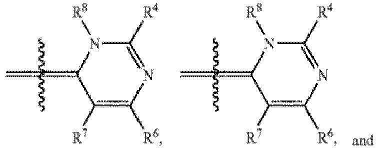 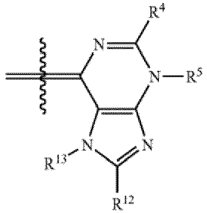 and
   wherein when v=1, Q is an heterocycle selected from the group of structures consisting of: 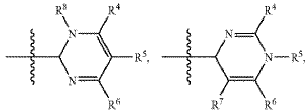 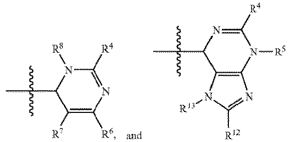 wherein R⁴, R⁵, R⁶, R⁷, R⁸, R¹², and R¹³ are independently selected from the group consisting of hydrogen, halogen, alkyl, cycloalkyl, heteroalkyl, heterocycloalkyl, alkenyl, polyalkenyl, alkynyl, polyalkynyl, alkenylalkynyl, alkylnitrilethio, aryl, heteroaryl, alkoxy, arylcarbonylthio, cycloheteroalkylcarbonylthio, dialkylaminoalkylcarbonylthio, dialkylamino, cycloalkylthio, cycloheteroalkylthio, trialkylammoniumalkylthio, trialkylammoniumalkylcarbonylthio, and nucleosidylthio, each of which may be optionally substituted; an acyclic heteroatom-containing moiety, a cyclic heteroatom-containing moiety, a BRIDGE-DYE, and a reactive group, each of which optionally includes a quaternary ammonium moiety.

Also described herein, at least one of the dsDNA binding dyes of the blend is a compound having the formula 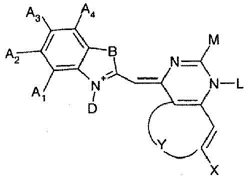 characterized in that either all of A1, A2, A3 and A4 are H or one of A1, A2, A3 and A4 is a substituent which is a halogenyl, and the others are H; B is selected from a group consisting of S, O, N-R, and C-(R)₂ wherein R is C₁-C₆-Alkyl; D is either an unsubstituted or a substituted C₁-C₆-alkyl; X is either H or a methoxy-group; Y is selected from a group consisting of S, O, N-R wherein R is C₁-C₆-Alkyl, and Z1-C=C-Z2, wherein Z1 and Z2 independently from each other are either H or a methoxy-group; L is either CH₃ or phenyl; and M is either phenyl or a substituted or unsubstituted C₁-C₁₈ amino-alkyl.

Also described herein, at least one of the dsDNA binding dyes of the blend is a compound having the formula 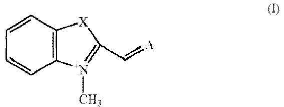 wherein A is 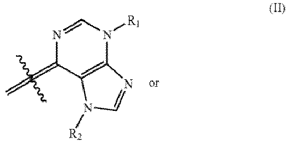 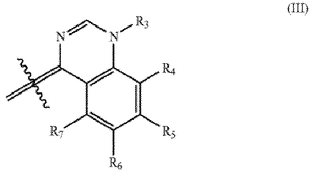 wherein X is O or S; R₁, R₂, R₃, R₄, R₅, R₆ and R₇ are independently hydrogen, halogen, thiol, optionally substituted alkyl, optionally substituted alkoxy, optionally substituted alkenyl, optionally substituted polyalkenyl, optionally substituted alkynyl, optionally substituted polyalkynyl, optionally substituted thioalkyl, optionally substituted aminoalkyl, optionally substituted aryl, optionally substituted heteroaryl and optionally substituted arylalkyl; R₁ and/or R₂ carry one or more positive charges such that the sum of the positive charge(s) on R₁ and R₂ is 1, 2 or 3; and R₃, and/or R₄, and/or R₅, and/or R₆ and/or R₇ carry one or more positive charges such that the sum of the positive charge(s) on R₃, R₄, R₅, R₆ and R₇ is 1, 2 or 3.

Also described herein, at least one of the dsDNA binding dyes of the blend is a compound having the formula 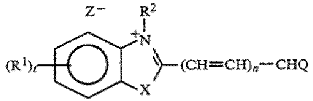 wherein each R¹ is independently H; or an alkyl group having from 1-6 carbons; or a trifiuoromethyl; or a halogen; or -OR⁸,- SR⁸ or -(NR⁸R⁹) where R⁸ and R⁹, which can be the same or different, are independently H; or alkyl groups having 1-6 carbons; or 1-2 allcyclic, heteroalicyclic, aromatic or heterouromatic nngs, containing 1-4 heteroatoms, wherein the hetero atoms are O, N or S; or R⁸ and R⁹ taken in combination are -(CH₂)₂-L-(CH₂-)₂-where L=a single bond, -O-, -CH₂-, or -NR₁₀-, where R¹⁰ is H or an alkyl group having 1-6 carbons; and t = 1-4; R² is an alkyl group having 1-6 carbons; X is O, S, Se or -NR¹⁵, where R¹⁵ is H or an alkyl group having 1-6 carbons; or X is CR¹⁶R¹⁷ where R¹⁶ and R¹⁷, which may be the same or different, are independently alkyl groups having 1-6 carbons, or R¹⁶ and R¹⁷ taken in combination complete a five or six membered saturated ring; n=0, 1 or 2; Z is a biologically compatible counterion; Q has the formula Q1 or Q2 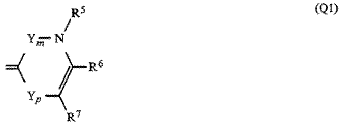 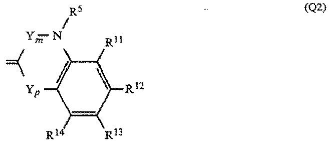 wherein Y is -CR³ = CR⁴-; p and m=0 or 1, such that p+m=t; R⁵ is an alkyl, alkenyl, polyalkenyl, alkynyl or polyailkynyl group having 1-6 carbons; or R⁵ is a OMEGA; R³, R⁴, R⁶ and R⁷, which may be the same or different, are independently H; or an alkyl, alkenyl, polyalkenyl, alkynyl or polyatlcynyl group having 1-6 carbons; or a halogen; or -OH, -OR⁸, -SR⁸, -(NR⁸R⁹); or -OSO₂R¹⁹ where R¹⁹ is alkyl having 1-6 carbons, or perfluoroalkyl having 1-6 carbons, or aryl; or an OMEGA; or R⁶ and R⁷, taken in combination are -(CH₂)ᵥ- where v=3 or 4, or R⁶ and R⁷ form a fused aromatic ring according to formula Q2; R¹¹, R¹², R¹³, and R¹⁴, which may be the same or different, are independently H, or an alkyl, alkenyl, polyalkenyl, alkynyl or polyalkynyl group having 1-6 carbons; or a halogen; or an OMEGA; or -OH, -OR⁸, -SR⁸, or -(NR⁸R⁹); OMEGA is a cyclohexyl, cyclohexenyl, morpholino, piperidinyl, naphthyl, phenyl, thienyl, benzothiazolyl, furanyl, oxazolyl, benzoxazolyl or pyridinyl that is unsubstituted or optionally substituted one or more times, independently, by halogen, alkyl, perfluoroalkyl, areinc, alkylamino, dialkylamino, alkoxy or carboxyalkyl, having 1-6 carbons, and that is attached as R³, R⁴, R⁵, R⁶, R⁷, R¹¹, R¹², R¹³, or R¹⁴ by a single bond; such that at least one of R³, R⁴, R⁵, R⁶, R⁷, R¹¹, R¹², R¹³, and R¹⁴, is an OMEGA, and, where more than one of R³, R⁴, R⁵, R⁶, R⁷, R¹¹, R¹², R¹³, and R¹⁴ is an OMEGA, each OMEGA is optionally the same or different; and such that when O has the formula Q1, n=O.

Also described herein, at least one of the dsDNA binding dyes of the blend is a compound having the formula 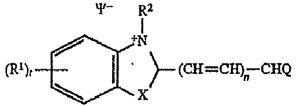 wherein each R¹ is independently H; or an alkyl group having from 1-6 carbons; an alkoxy group having from 1-6 carbons; or a trifluoromethyl; or a halogen; and t=1-4; R² is an alkyl group having 1-6 carbons; X is O, S, Se or NR¹⁵, where R¹⁵ is H or an alkyl group having 1-6 carbons; or X is CR¹⁶R¹⁷ where R¹⁶ and R¹⁷, which may be the same or different, are independently alkyl groups having 1-6 carbons, or R¹⁶ and R¹⁷ taken in combination complete a five or six membered saturated ring; n=0, 1 or 2; ψ⁻ is a biologically compatible counterion; Q has the formula Q1 or Q2 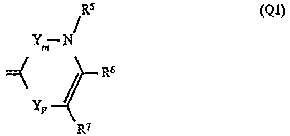 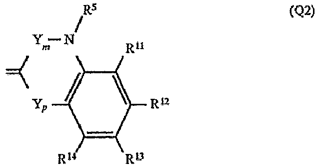
wherein Y is -CR³=CR⁴-; p and m=0 or 1, such that p+m=l; R⁵ is an alkyl, alkenyl polyalkenyl, alkynyl or polyalkynyl group having 1-6 carbons; or R⁵ is a cyclic substituent that is a substituted or unsubstituted aryl or heteroaryl; or a substituted or unsubstituted cycloalkyl having 3-10 carbons; or R⁵ is a TAIL;
R³, R⁴, R⁶ and R⁷, which may be the same or different, are independently H; or an alkyl, alkenyl. polyalkenyl, alkynyl or polyalkynyl group having 1-6 carbons; or a halogen; or a substituted or unsubstituted aryl or heteroaryl; or a substituted or unsubstituted cycloalkyl having 3-10 carbons; or
-OR⁸, -SR⁸, -(NR⁸R⁹); or -OSO₂R¹⁹; or a TAIL; where R⁸ and R⁹, which can be the same or different, are independently H; or alkyl groups having 1-6 carbons; or 1-2 alicyclic or aromatic rings; or R⁸ and R⁹ taken in combination are -(CH₂)₄- or -(CH₂)₅- to give a 5 or 6 membered ring; and where R¹⁹ is alkyl having 1-6 carbons, or perfluoroalkyl having 1-6 carbons, or aryl; or R⁶ and R⁷, taken in combination are -(CH₂)ᵥ-where v=3 or 4, or R⁶ and R⁷ form a fused aromatic ring according to formula Q2; R¹¹, R¹², R¹³, and R¹⁴, which may be the same or different, are independently H; or an alkyl, alkenyl, polyalkenyl, alkynyl or polyalkynyl group having 1-6 carbons; or a halogen; or a TAIL; or -OH, -OR⁸, -SR⁸, or -(NR⁸R⁹); TAIL is a heteroatom-containing moiety having the formula LINK-SPACER-CAP; wherein LINK is a single covalent bond, -O-, -S-, or -NR²⁰-; where R²⁰ is H, a linear or branched alkyl having 1-8 carbons, or R²⁰ is -SPACER'-CAP'; SPACER and SPACER', which may be the same or different, are covalent linkages, linear or branched, cyclic or heterocyclic, saturated or unsaturated, each having 1-16 nonhydrogen atoms selected from the group consisting of C, N, P, O and S, such that the linkage contains any combination of ether, thioether, amine, ester, amide bonds; or single, double, triple or aromatic carbon-carbon bonds; or phosphorus-oxygen, phosphorus-sulfur bonds, nitrogen-nitrogen or nitrogen-oxygen bonds; or aromatic or heteroaromatic bonds; CAP and CAP', which may be the same or different, are -O-R²¹, -S-R²¹,-NR²¹R²², or -N+R²¹R²²R²³ ψ⁻; wherein R²¹, R²², and R²³ are independently H, or a linear or branched alkyl or cycloalkyl having 1-8 carbons, optionally further substituted by hydroxy, alkoxy having 1-8 carbons, carboxyalkyl having 1-8 carbons, or phenyl, where phenyl is optionally further substituted by halogen, hydroxy, alkoxy having 1-8 carbons, aminoalkyl having 1-8 carbons, or carboxyalkyl having 1-8 carbons; or, one or more of R²¹, R²² and R²³, taken in combination with SPACER or SPACER' or R²⁰ forms a 5- or 6-membered aromatic, heteroaromatic, alicyclic or heteroalicyclic ring, the heteroatoms selected from O, N or S; where ψ⁻ is a biologically compatible counterion; or CAP and CAP' are independently 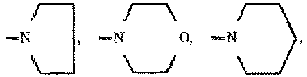 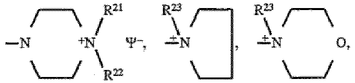 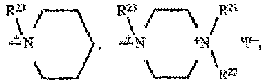 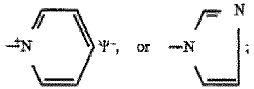 where R²¹, R²² and ψ⁻ are as defined previously; such that at least one of R³, R⁴, R⁵, R⁶, R⁷, R¹¹, R¹², R¹³, and R¹⁴ is a TAIL; and when R⁵ is a TAIL, LINK is a single bond; and where only R⁵ is a TAIL, R³ or R⁴ is not hydrogen; and, where more than one of R², R³, R⁴, R⁵, R⁶, R⁷, R¹¹, R¹², R¹³, and R¹⁴ is a TAIL, each TAIL is optionally the same or different.

Dye saturation is a consideration when selecting dye blends, because a dye with a high percent saturation performs better in HRM analysis. Dye saturation is defined as the fluorescence at a certain dye concentration, typically the concentration usable for qPCR and typically 1X, as a percentage of the maximum fluorescence obtained when dsDNA is titrated with an increasing dye concentration. When a predetermined concentration of dsDNA is titrated with increasing concentrations of a dsDNA-binding dye, the fluorescence increases until all the dye-binding sites on the dsDNA are occupied. At this point, the fluorescence plateaus or decreases because the dsDNA is saturated with dye. Individual dyes are primarily designed for either qPCR or HRM; using a dye blend provides optimal properties for both qPCR and HRM.

The disclosed dye blends have properties that each dye alone does not provide. A blend of dsDNA binding dyes provided improved properties compared with the individual dyes alone, e.g., higher fluorescence and the same or lower Cq compared to each dye alone. In embodiments, dye blends were used at a concentration of the individual dyes that was generally lower than would be used if the dyes were used alone. Using a relatively lower dye concentrations is generally less inhibitory to other processes, e.g., PCR. However, the dyes in the dye blends provided an enhanced effect on dsDNA saturation, where saturation is increased using dye concentrations that were not inhibitory to qPCR. For example, a dye blend may contain two dyes, each at a concentration that, if used alone, would provide about 60% DNA saturation, but the dye blend provided about 90% or greater DNA saturation. Thus, the dye blends showed a synergistic effect by improving performance beyond the performance of each dye alone. This synergistic effect may reflect different binding modes on the dsDNA by the different dyes forming the dye blend.

Without being bound to a specific theory, the improved characteristics of the described dsDNA binding dye blends may increase the design space for dye concentration in a mix for HRM and qPCR. Using the blend of dsDNA binding dyes permits fluorescence maxima to be achieved at a lower concentration of each dye than using each individual dye alone, and at a concentration that is less inhibitory to DNA polymerase enzymes. The dye blends improved the fluorescence characteristics of qPCR and HRM, and also overcame the problems of qPCR inhibition associated with saturating concentrations of dsDNA binding dyes.

In one embodiment, a dye blend of two dyes blends was as follows: structurally similar dyes V11-02190 and V11-03001, and structurally distinct dyes V11-02190 and Eva Green. 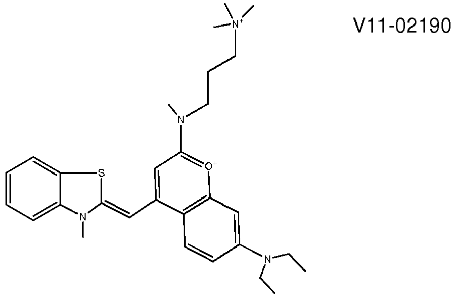 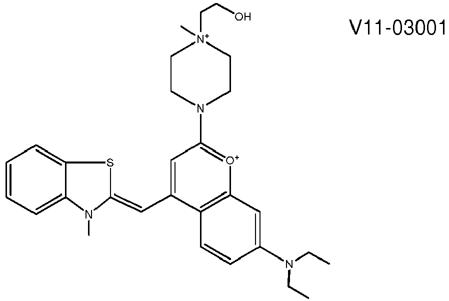 Although the manufacturer does not provide the exact structure of Eva Green@, it is thought to be two homo-monomers linked by a bridge.

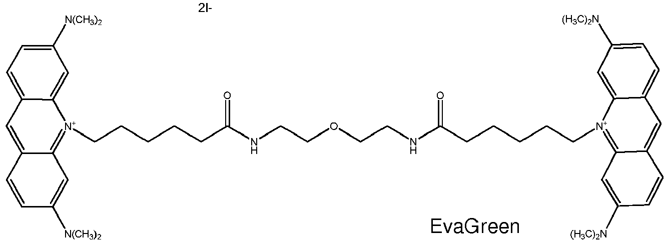

The performance of the dye blend in qPCR was compared in triplicate qPCR reactions across a series of 10-fold dilutions of human genomic DNA (hgDNA; Roche, Cat#. 1691112) from 50 ng - 50 pg per reaction using a glyceraldehyde phosphate dehydrogenase (GAPDH) 94 assay GAPDH 94 F 5' ACAGTCAGCCGCATCTTCTT 3' (SEQ ID. NO: 1); GAPDH 94 R 5' ACGACCAAATCCGTTGACTC 3' (SEQ ID. NO: 2); final concentration each primer 400 nM).

PCR reactions, 15 µl, comprised 10 mM Tris pH 8.2; 500 mM KCI; 0.01 mM EDTA ; 3 mM MgCl2; 200pM of each dNTP (Thermo Fisher Scientific); 0.01% Tween 20; 0.005 U/pl Thermo-start DNA polymerase (Thermo Fisher Scientific). Molecular grade reagents were from Sigma unless otherwise stated.

A blend of two structurally similar dyes and two structurally dissimilar dyes were selected as representative dyes. The two structurally similar dyes were V11-02190 and V11-03001, as disclosed in PCT/US08/78277. The two structurally dissimilar dyes were V11-02190 and Eva Green®. Each dye in the respective dye blend was included in the reaction mixtures at the concentrations described. Each dye was added alone at each of the different concentrations as control reactions. All of the described dyes are non-specific dsDNA binding dyes. Single dyes were included as controls at 0.5X to 2X optimum concentration of each dye, which was 8 µM V11-02190, 1 µM V11-03001, 1 X Eva Green. Reactions were cycled on a Roche LC480 thermal cycler using a 384 well block with the following conditions: 95°C for 15 minutes, followed by 50 cycles of 95°C for 15 seconds, 60°C for 20 seconds, 72°C for 20 seconds during which fluorescence data was acquired, following which a melting curve was acquired at 65°C-95°C. Fluorescence data were generated by exciting the dye(s), whether a single dye or a dye blend, at a single excitation wavelength and measuring emission at a single emission wavelength. As is known in the art, a single excitation or emission wavelength may comprise a narrow range of excitation and/or emission wavelengths, such as a range of about 1 nm to about 20 nm.

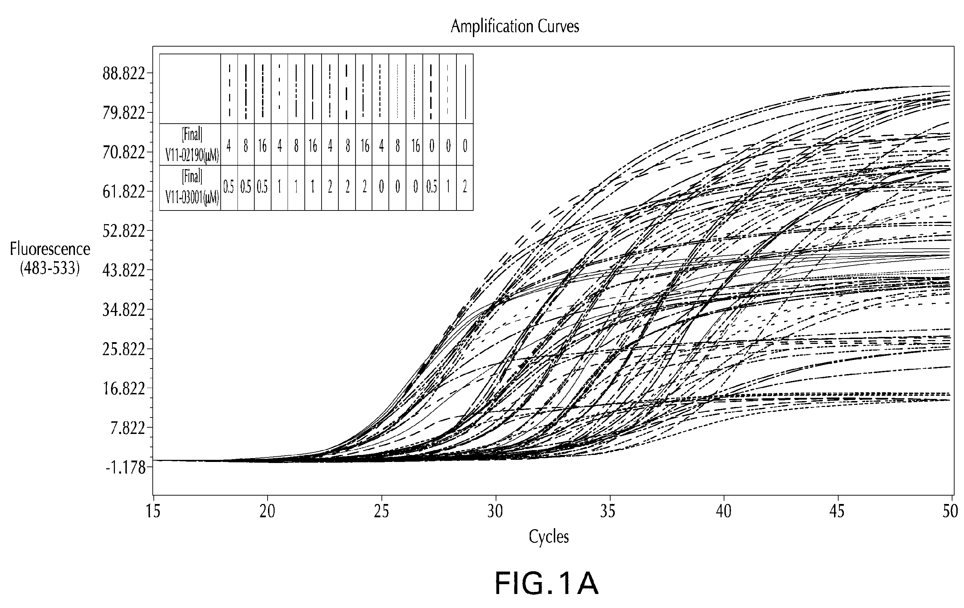

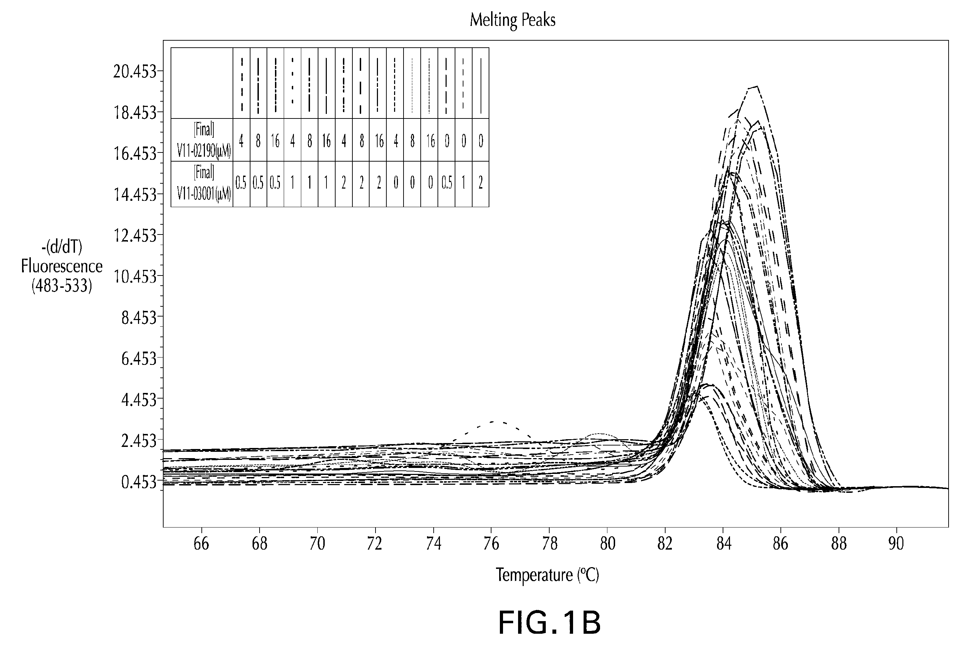

A blend of V11-02190 and V11-03001, at various concentrations, was compared with each dye separately, at various concentrations, in generating either an amplification curve or a melting peak, using the above-described method and as shown in FIGS. 1A, 1B. The colors and traces indicate the presence and concentration of each dye in the sample according to the following table, the colors serving as a key to the traces in FIGS. 1A-1B.

| | | | | | | | | | | | | | | | |
|---|---|---|---|---|---|---|---|---|---|---|---|---|---|---|---|
| **[Final] V11-02190 (µM)** | 4 | 8 | 16 | 4 | 8 | 16 | 4 | 8 | 16 | 4 | 8 | 16 | 0 | 0 | 0 |
| [**Final**] **V11-03001 (µM)** | 0.5 | 0.5 | 0.5 | 1 | 1 | 1 | 2 | 2 | 2 | 0 | 0 | 0 | 0.5 | 1 | 2 |

Some dye blends showed improved characteristics compared with individual dye conditions. Tₘ peaks, as shown in FIG. 1B, from 50 pg human genomic DNA (hgDNA) reactions showed excellent specificity at all dye concentrations. This can be seen by improved end-point fluorescent signals and lower calculated Cq values, indicative of better PCR amplification efficiency.

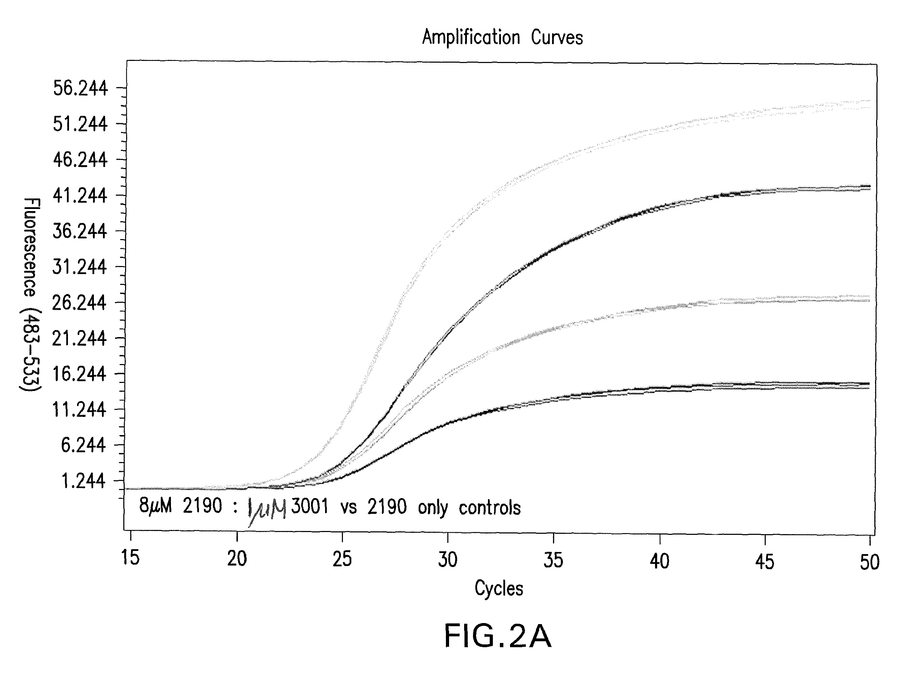

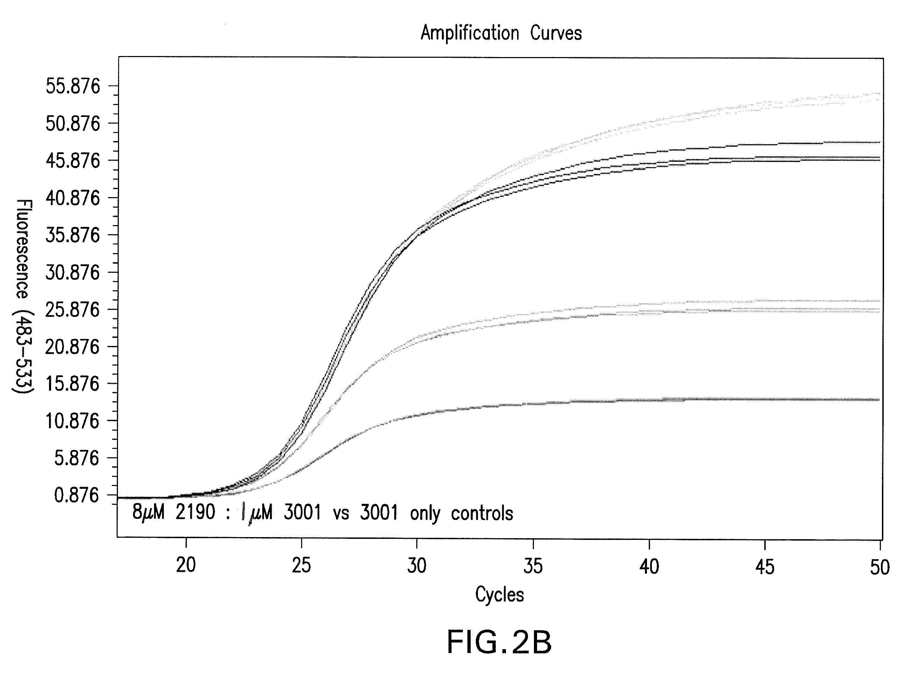

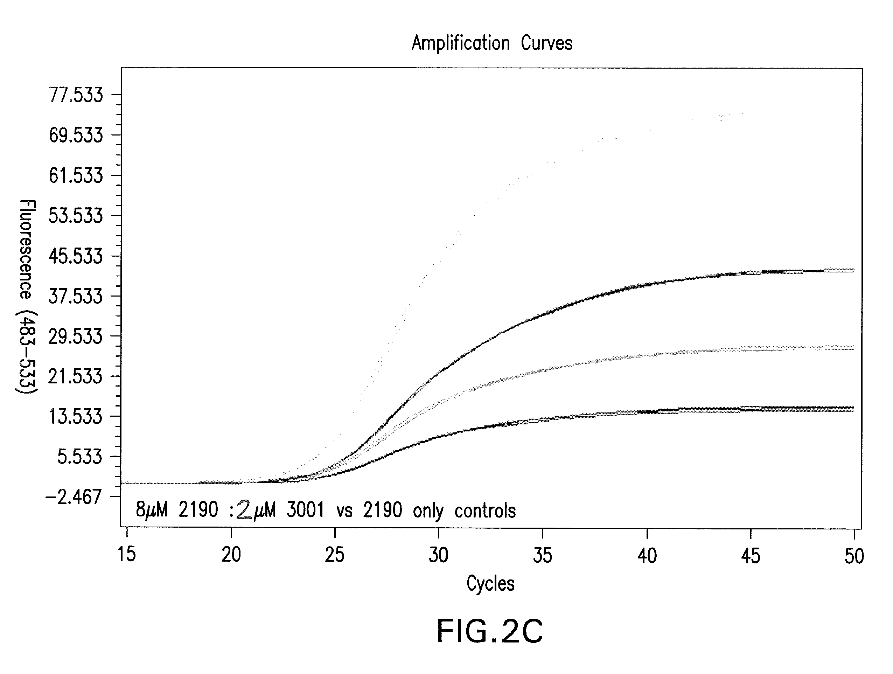

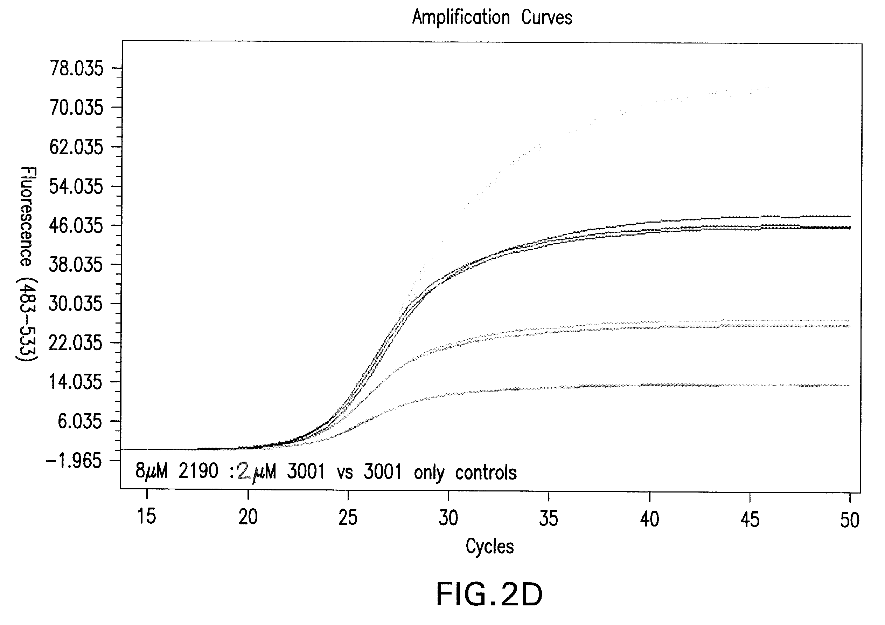

FIGS. 2A, 2B compare results from a blend of V11-02190 (8 µM) and V11-03001 (1 µM) with either V11-02190 alone at 4 µM, 8 µM, and 16 µM (FIG. 2A), or with V11-03001 alone at 0.5 µM, 1 µM, and 2 µM (FIG. 2B). FIGS. 2C and 2D compare results from a blend of V11-02190 (8 µM) and V11-03001 (2 µM) with either V11-02190 alone at 4 µm, 8 µm, and 16 µm (FIG. 2C), or with V11-03001 alone at 0.5 µM, 1 µM, or 2 µM (FIG. 2D). Colors of the various traces in FIGS. 2A-2D indicate the presence and concentration of each dye V11-02190 and/or V11-03001 in the sample, according to the following table, the colors serving as a key to the traces in FIGS 2A-2D.

| | | | | | | | | |
|---|---|---|---|---|---|---|---|---|
| **[Final] V11-02190 (µM)** | 8 | 8 | 4 | 8 | 16 | 0 | 0 | 0 |
| **[Final] V11-03001 (**µ**M)** | 1 | 2 | 0 | 0 | 0 | 0.5 | 1 | 2 |

Several blends showed higher endpoints and lower Cq than single dyes alone (controls) even at increased concentrations, e.g.. 8 µM V11-2190 with either 1 µM V11-3001 or 2 µM V11-3001.

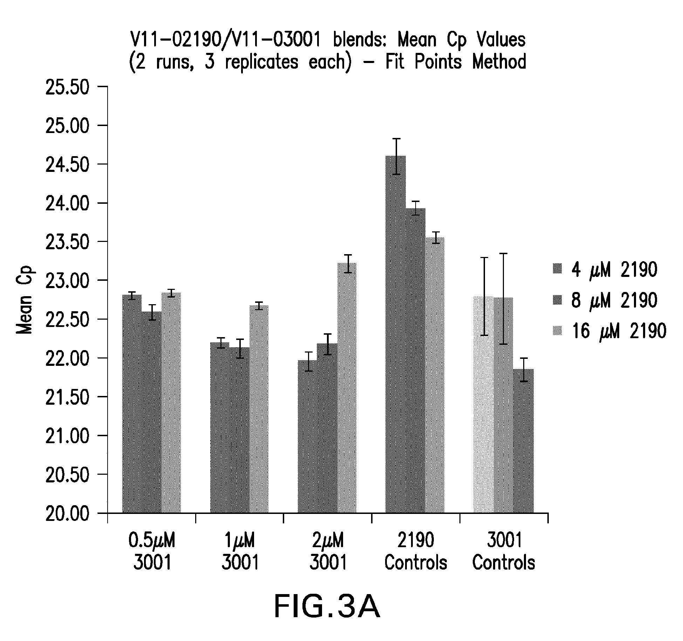

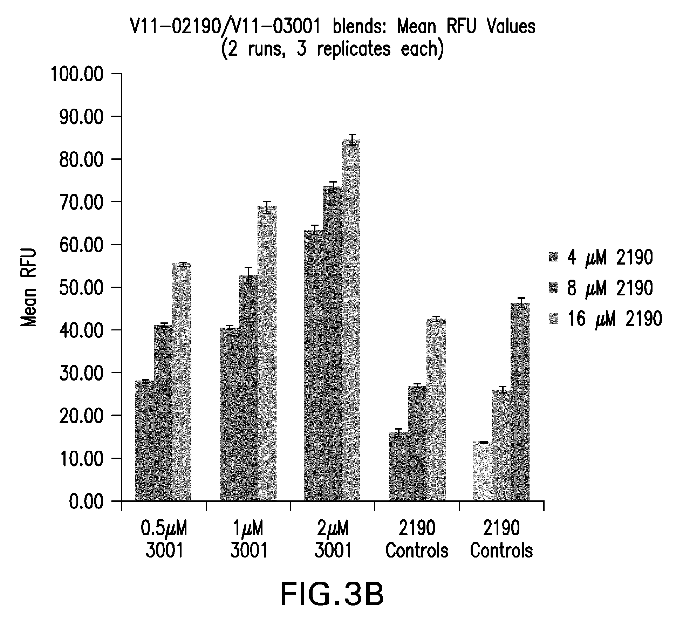

The amplification curves comparing V11-02190/V11-03001 blends with single dyes were analyzed to determine mean Cq using the fit points/threshold/Cq call method (which are user-defined on the Roche Lightcycler 480 software) as shown in FIG. 3A, and endpoint fluorescence based on mean relative maximum fluorescence units (RFU) as shown in FIG. 3B. A dye with a low Cq is useful in qPCR. A dye with a high RFU is useful in HRM analysis. A dye blend with a low Cq and high RFU is useful for both qPCR and HRM analysis. In FIG. 3A and 3B, the indicated concentration of V11-03001 was combined with either 4 µM V11-02190 (blue bars), 8 µM V11-02190 (orange bars), or 16 µM V11-02190 (green bars), and compared with V11-02190 alone (V11-2190 controls) or V11-03001 alone (V11-3001 controls) at either 0.5 µm (light bar), 1 µM (medium bar), or 2 µM (dark bar).

Using the fit points/threshold/C_{q} call method, dye blends that produced the combined highest RFU and lowest Cq results were 8 µM V11-02190 and 2 µM V11-0301; and 8 µM V11-02190 and 1 µM V11-03001.

The results of the V11-02190 and V11-03001 dye blend showed that blending dyes increased endpoint fluorescence compared with individual dyes alone. For example, even using an increased concentration (16 µM) of dye V11-02190 alone, there was a lower endpoint fluorescence when compared to a dye blend containing 8 µM V11-02190 and 2 µM VI 1-03001. Dye blends also improved Cq compared to Cq with individual dyes alone. For example, a dye blend of V11-02190 and V11-03001 showed lower Cq than an equivalent concentration of V11-02190 alone and lower or approximately equivalent concentration of V11-03001 alone. The dye blends provided a compromise and provided properties that neither dye alone could provide. Cq calculated using the second derivative method appeared to penalize dye blends with higher endpoints due to the steeper amplification curve; Cq calculated using fit points/threshold/C_{q} call method provided more representative results based on analysis of the amplification plots. Thus, the dye blends avoided qPCR inhibition from increasing a single dye on its own, while providing improved saturation data.

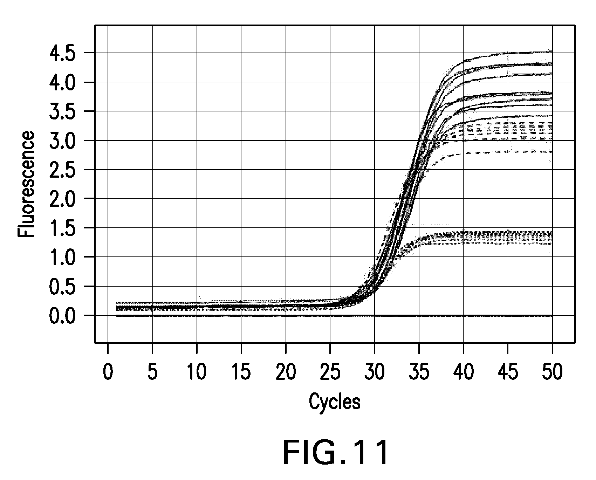

In addition to dye blends exhibiting improved properties compared to single dyes, the dye blends also exhibited minimal PCR inhibition, as demonstrated by the lack of increase in Cq value, and only at the highest concentrations of each dye in the blend. For example, dye V11-02190 at a concentration of 16 µM exhibited significant PCR inhibition; V11-03001 at a concentration of 2 µM exhibited significant PCR inhibition, shown in FIG. 12, and in FIG. 11 that shows 1XLC Green (dark solid line); 0.25X LC Green and 20 µM V11-02190 (dark dashed line); 20uM V11-02190 (dark dotted line).

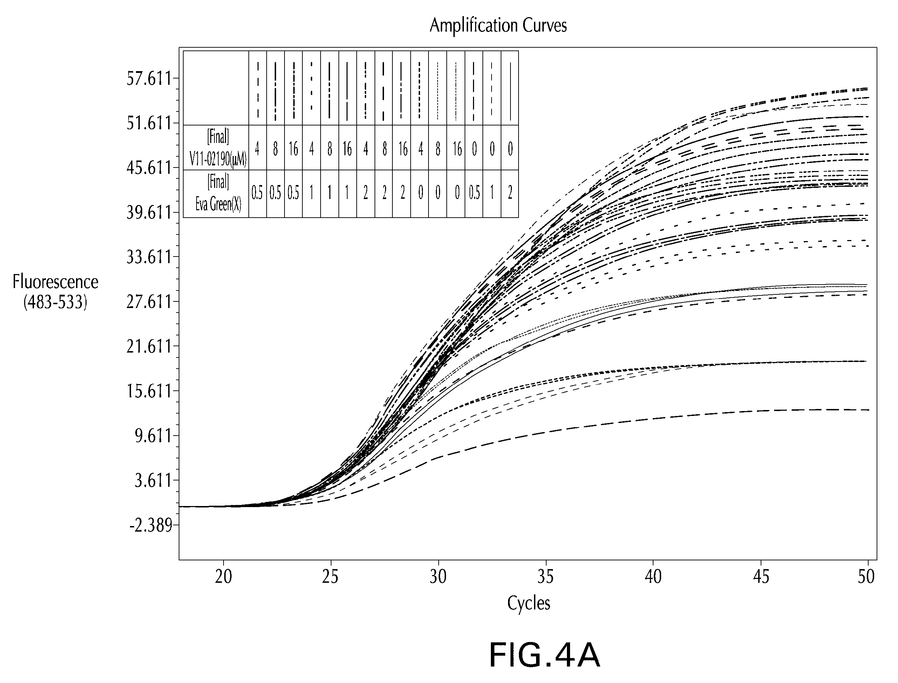

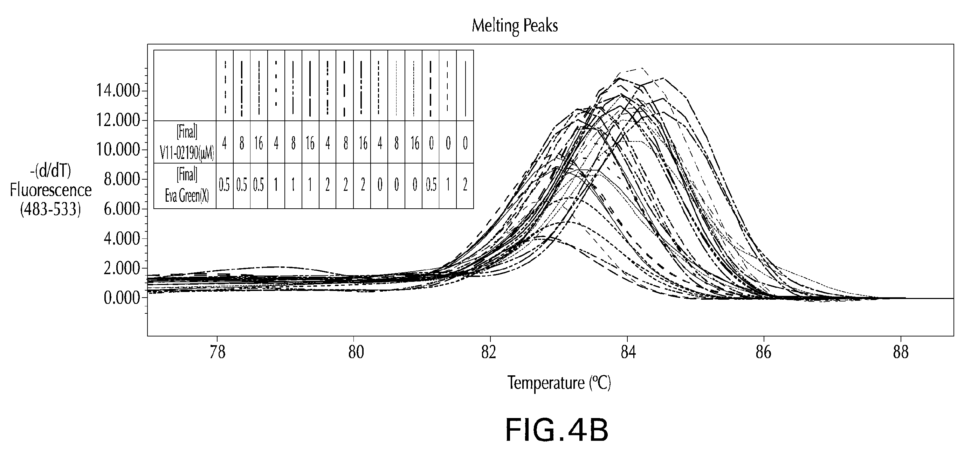

A dye blend of V11-02190 and a commercially available dye Eva Green@ (Biotium) were evaluated at various concentrations, and the blend was compared with each dyes alone at various concentrations, in both qPCR and melting curve analysis using the methods described above. The results are shown in FIGS. 4A, 4B; which indicates the presence and concentration of V11-02190 and/or Eva Green@ in the sample, with X indicating use at either 1X or other concentration from the manufacturer.

| | | | | | | | | | | | | | | | |
|---|---|---|---|---|---|---|---|---|---|---|---|---|---|---|---|
| **[Final] V11-02190 (µM)** | 4 | 8 | 16 | 4 | 8 | 16 | 4 | 8 | 16 | 4 | 8 | 16 | 0 | 0 | 0 |
| **[Final] Eva Green (X)** | 0.5 | 0.5 | 0.5 | 1 | 1 | 1 | 2 | 2 | 2 | 0 | 0 | 0 | 0.5 | 1 | 2 |

Some dye blends showed improved characteristics compared with individual dye conditions. Tm peaks, as shown in FIG. 4B, from 50 pg hgDNA reactions showed excellent specificity at all dye concentrations. This can be seen by improved end-point fluorescent signals and lower calculated Cq values, indicative of better PCR amplification efficiency.

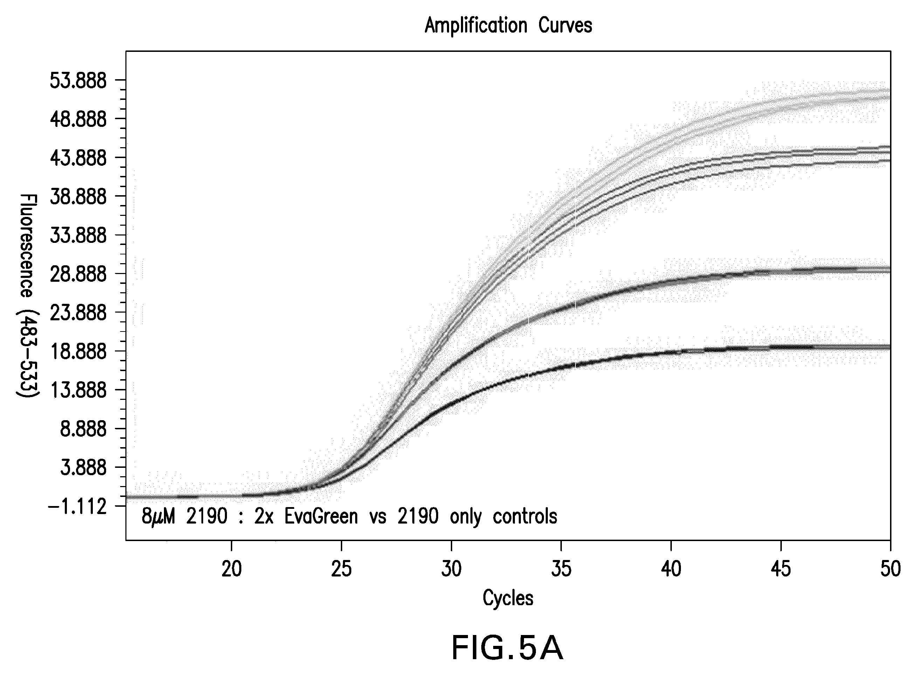

Specific dye blends were further analyzed, and the data are shown in FIGS. 5A-5D, where the colors of the various traces shown in FIG. 5A-5D indicate the presence and concentration of V11-02190 and/or Eva Green@ in the sample, according to the following table, the colors serving as a key to the traces in FIGS 5A-5D.

| | | | | | | | | |
|---|---|---|---|---|---|---|---|---|
| **[Final] V11-02190 (µM)** | 16 | 8 | 4 | 8 | 16 | 0 | 0 | 0 |
| **[Final] Eva Green (X)** | 1 | 2 | 0 | 0 | 0 | 0.5 | 1 | 2 |

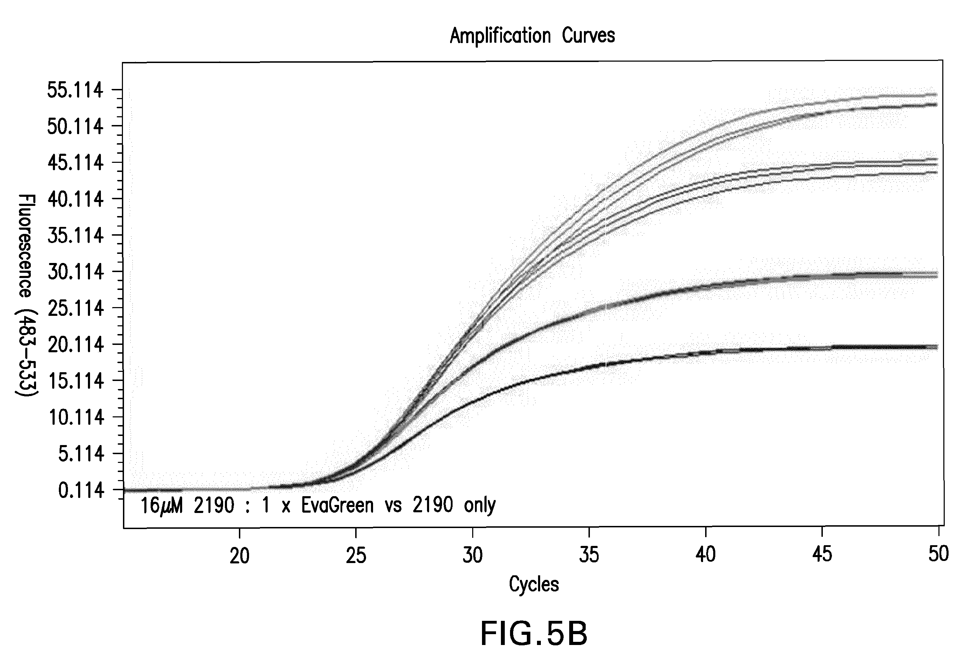

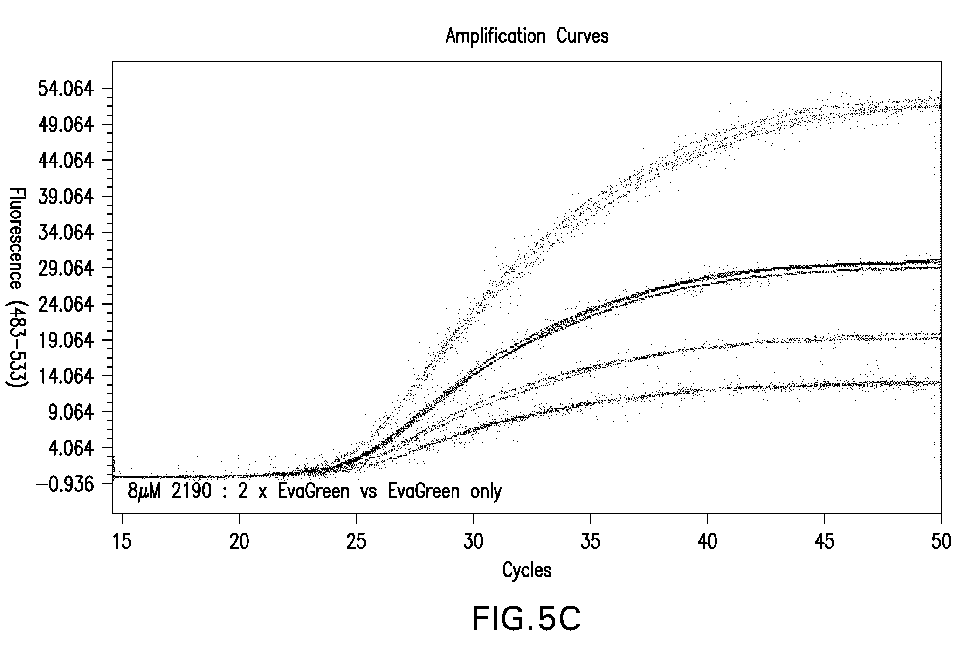

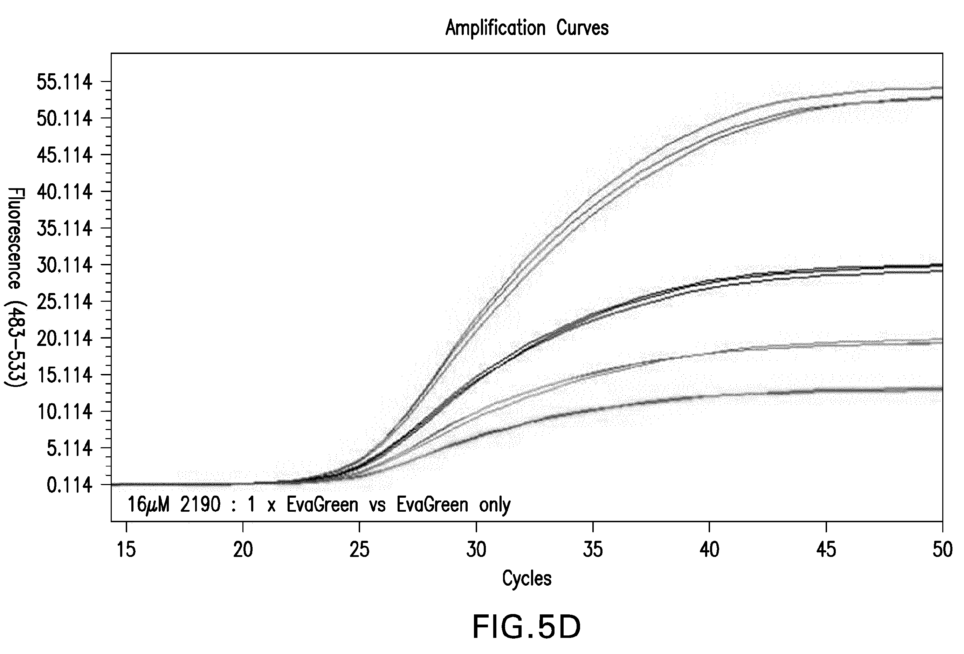

FIGS. 5A, 5B compare results from a blend of V11-02190 (8 µm) and Eva Green@ (2X) (FIG. 5A), or a blend of V11-02190 (16 µm) and Eva Green® (1X) (FIG. 5B), with V11-02190 alone at either 4 µm, 8 µm, or 16 µm. FIGS. 5C, 5D compare results from a blend of V11-02190 (8 µm) and Eva Green® (2X) (FIG. 5C), or a blend of V11-02190 (16 µm) and Eva Green® (1X) (FIG. 5D), with Eva Green® alone at either 0.5X, 1X, or 2X. As shown in FIGS. 5A-5D, several blends showed higher endpoint and lower Cq than single dyes alone (controls), even at increased concentrations. This is indicated by the curves' higher endpoint because the plateau is at higher RFU value and lower Cq because the curve shifts slightly to the left. Amplification was not inhibited and saturated increased.

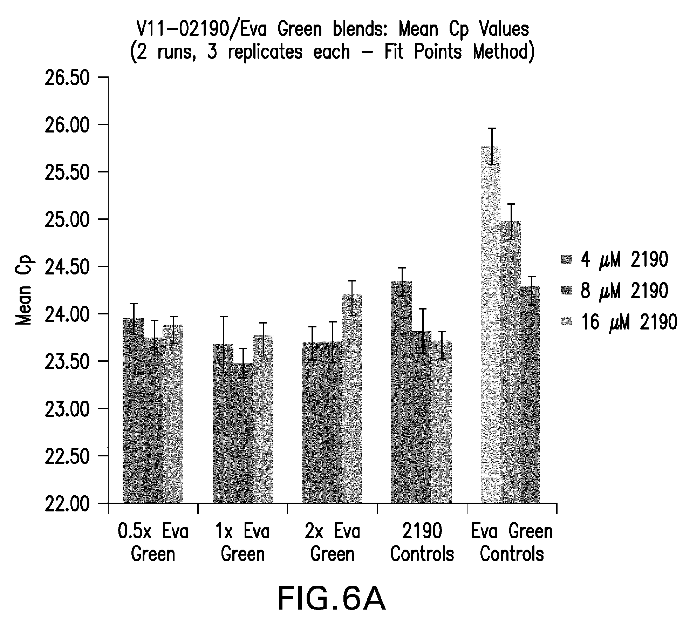

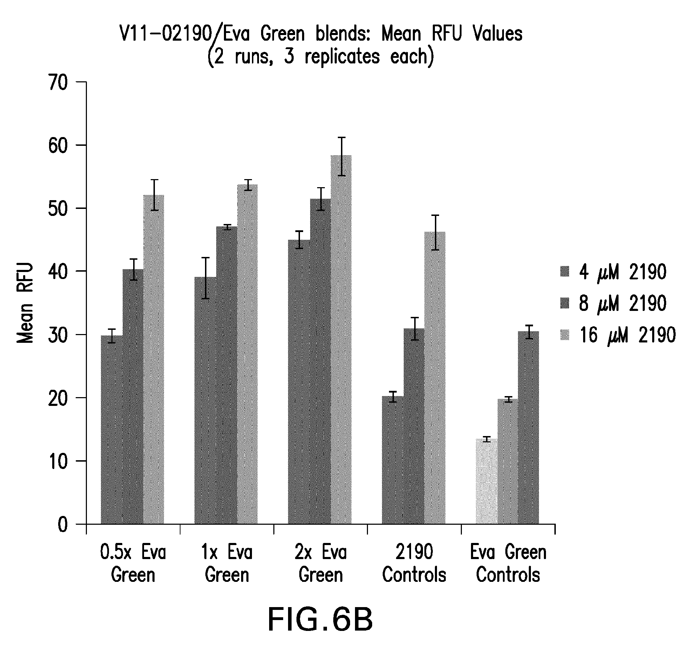

The amplification curves comparing a blend of V11-02190 and Eva Green@ with single dyes alone were analyzed to determine mean Cq, using the fit points/threshold/C_{q} call method, as shown in FIG. 6A, and endpoint fluorescence based on mean maximum relative fluorescence units (RFU), as shown in FIG. 6B. In FIGS, 6A, 6B, the indicated concentration of Eva Green® was combined with either 4 µm V11-02190 (blue bars), 8 µm V11-02190 (orange bars), or 16 µm V11-02190 (green bars), and compared with V11-02190 alone (V11-02190 controls) or Eva Green® alone (Eva Green controls) at either 0.5X (light bar), 1X (medium bar), or 2X (dark bar).

Using the fit points/threshold/Cq call method, dye blends that produced the combined highest RFU and lowest Cq results were 8 µM V11-02190 and 2X Eva Green@; 16 µM V11-02190 and 0.5X Eva Green®; and 16 µM V11-02190 and 1X Eva Green®. The results showed that blending dyes increased endpoint fluorescence compared with individual dyes alone, and improved Cq compared with individual dyes alone (V11-02190 control, Eva Green® control), see FIGS. 11, 12.

Dye saturation of selected dye blends from qPCR was compared to the individual dyes alone as an indication of potential performance in HRM analysis. Higher dye saturation provides less dye redistribution to non-denatured regions of the DNA during melting and thus results in higher melt sensitivity. To evaluate dye saturation, triplicate 25 µL samples were evaluated that contained 10 mM Tris pH 8.2; 500 mM KCI; 0.01 mM EDTA; and 10ng/ µl human genomic DNA (Roche cat#. 1691112). Each dye blend was added at the following concentrations: 20X, 10X, 5X, 2.5X, IX, 0.5X, 0.25X, 0.I X, and 0.01X; 1X is the concentration previously tested in qPCR. Fluorescence measurements were determined on BMG FluoSTAR Galaxy, using 485 nm excitation and 520 nm emission filters blanked using 10 mM Tris pH 8.2; 500 mM KCI; 0.01 mM EDTA buffer. Fluorescence values of control reactions (no dsDNA added) were subtracted from reactions containing dsDNA to produce fluorescence values normalized to remove any background fluorescence from unbound dye.

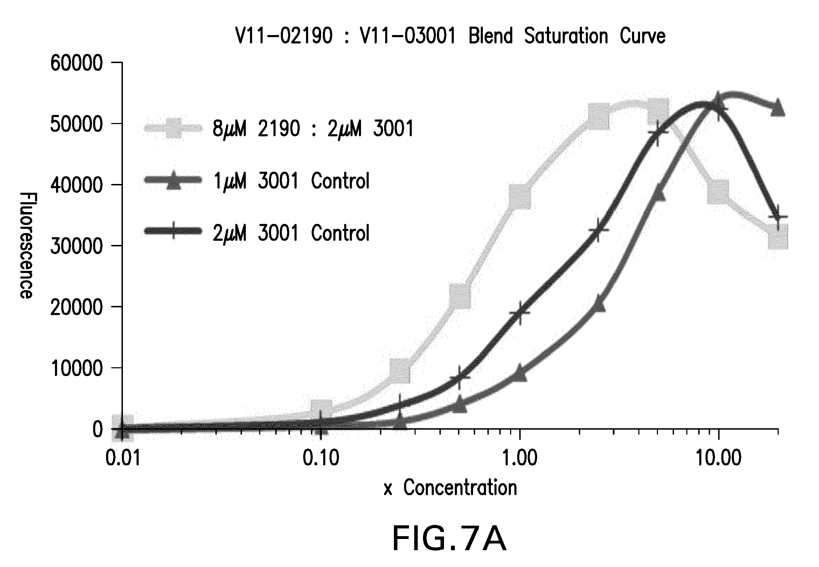

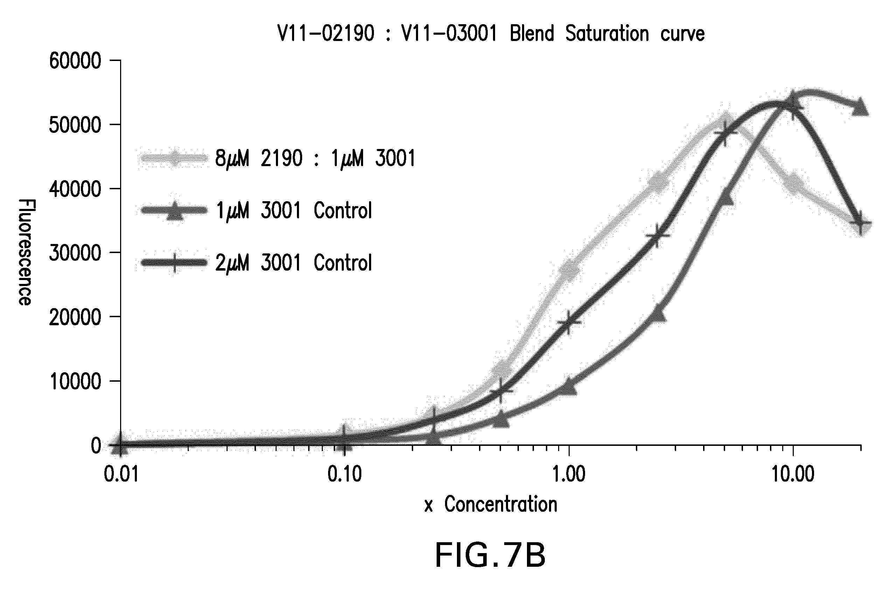

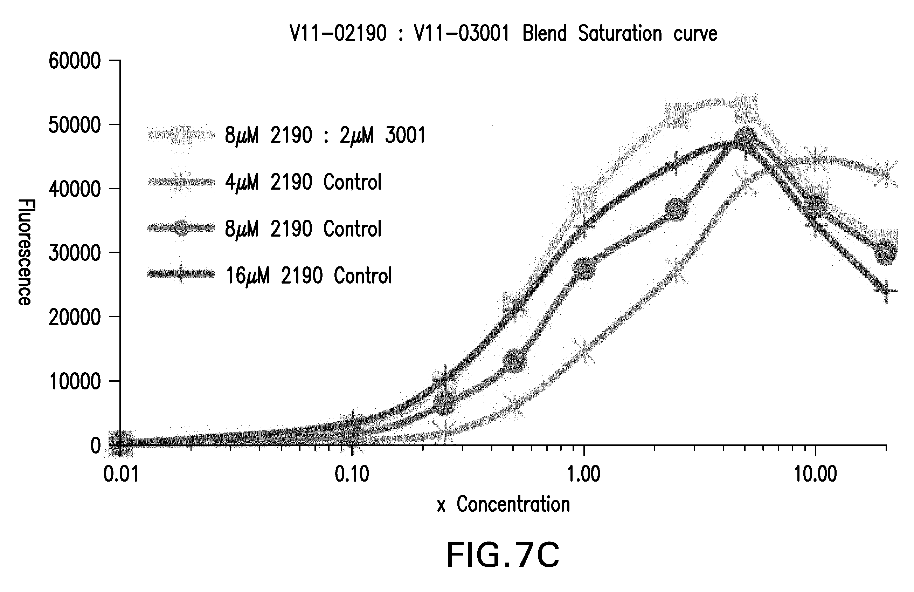

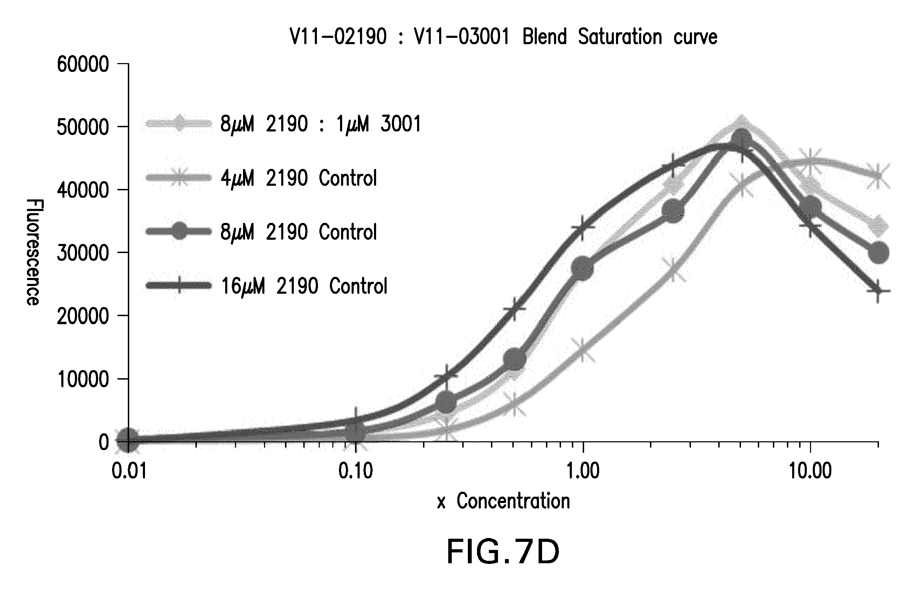

FIGS. 7A-7D shows saturation curves for dye blends of V11-02190 and V11-03001, compared to each dye alone, at various concentrations. FIG. 7A compares a dye blend of 8 µM V11-02190 and 2 µM V11-03001 (green squares), with V11-03001 alone at a concentration of either 1 µM (blue triangles) or 2 µM (purple cross). FIG. 7B compares a dye blend of 8 µM V11-02190 and 1 µM V11-03001 (blue diamonds), with V11-03001 at a concentration of either 1 µM (blue triangles) or 2 µM (purple cross). FIG. 7C compares a dye blend of 8 µM V11-02190 and 2 µm V11-03001 (green squares), with V11-02190 at a concentration of 4 µM (light pink double-cross), 8 µM (red circles), or 16 µM (red cross). FIG. 7D compares a dye blend of 8 µM V11-02190 and 1 µM V11-03001 (blue diamonds), with V11-02190 at 4 µM (light pink double-cross), 8 µM (red circles), or 16 µM (red cross).

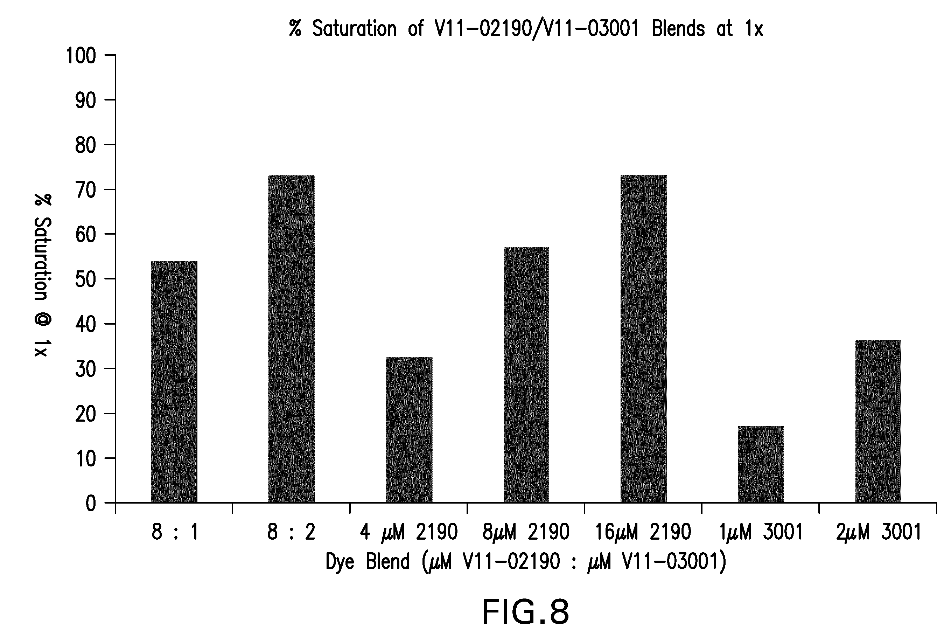

Dye blend 8 µM V11-02190 and 2 µM V11-03001 showed increased dye saturation, compared with V11-03001 controls, and a smaller increase compared with V11-02190 controls. Selected results from FIGS. 7A-7D were expressed in FIG. 8 as percent saturation at 1X. Dye blend 8µM V11-02190 and 2 µM V11-03001 showed increased saturation compared with V11-03001 controls and a smaller increase compared with V11-02190 controls. Dye blend 8 µM V11-02190 and 2 µM V11-03001 provided the highest saturation at 1X, about 73%. Improvements in dye saturation were modest and did not appear to be additive, indicating that dyes in the dye blends likely had similar modes of binding to dsDNA and competed for dsDNA binding sites.

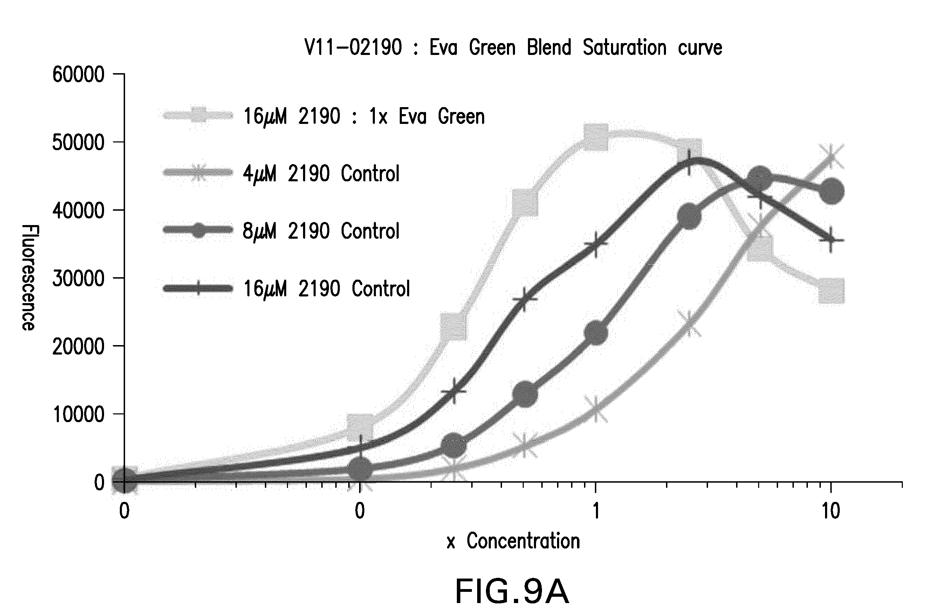

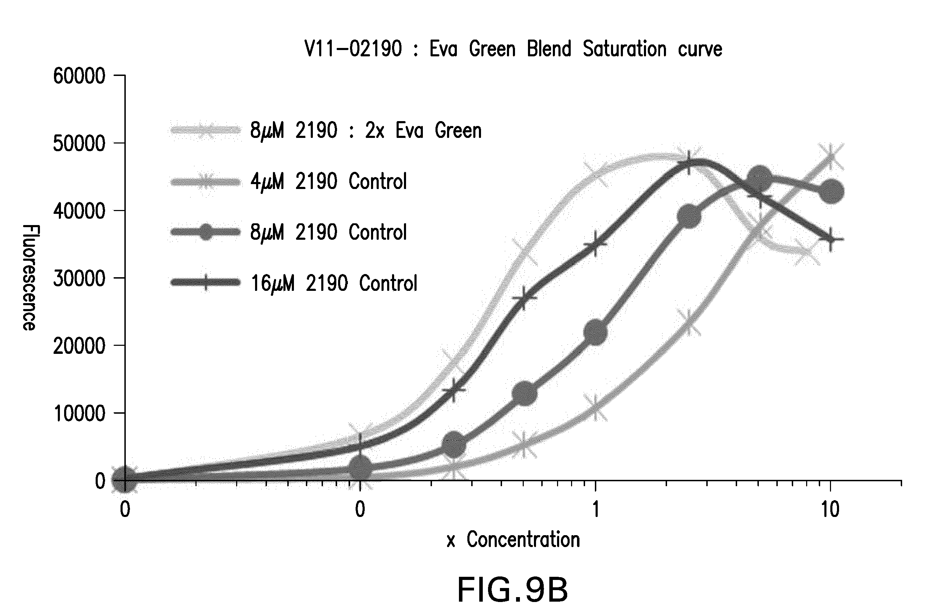

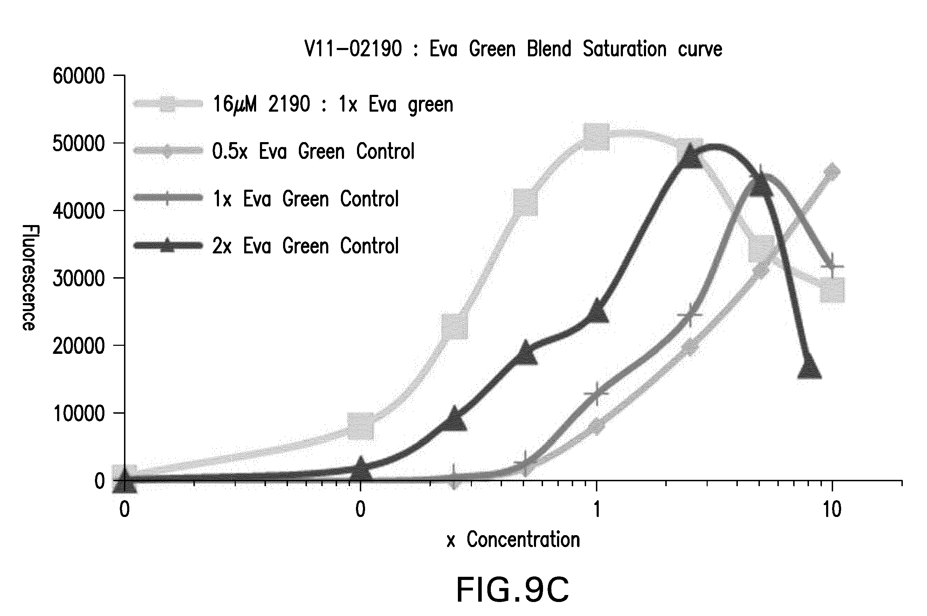

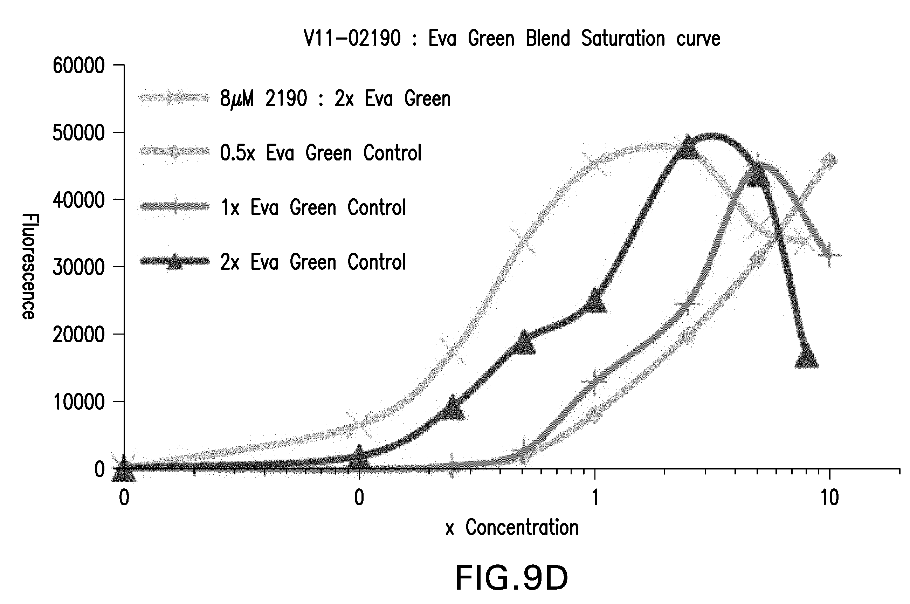

FIGS. 9A-9D show saturation curves for dye blends V11-02190 and Eva Green@, compared to each dye alone, at various concentrations. FIG. 9A compares a dye blend of 16 µM V11-02190 and 1X Eva Green® (green squares), with V11-02190 alone at a concentration of 4 µM (light pink double cross), 8 µM (red circles), and 16 µM (red cross). FIG. 9B compares a dye blend of 8 µM V11-02190 and 2X Eva Green@ (blue cross), with V11-02190 at a concentration of 4 µM (light pink double cross), 8 µM (red circles), and 16 µM (red cross). FIG. 9C compares a dye blend of 16 µm V11-02190 and 1X Eva Green® (green squares), with Eva Green® at a concentration of 0.5X (green diamonds), 1X (green cross), and 2X (green triangles). FIG. 9D compares a dye blend of 8 µM V11-02190 and 2X Eva Green® (blue cross), with Eva Green® at a concentration of 0.5X (green diamonds), 1X (green cross), and 2X (green triangles).

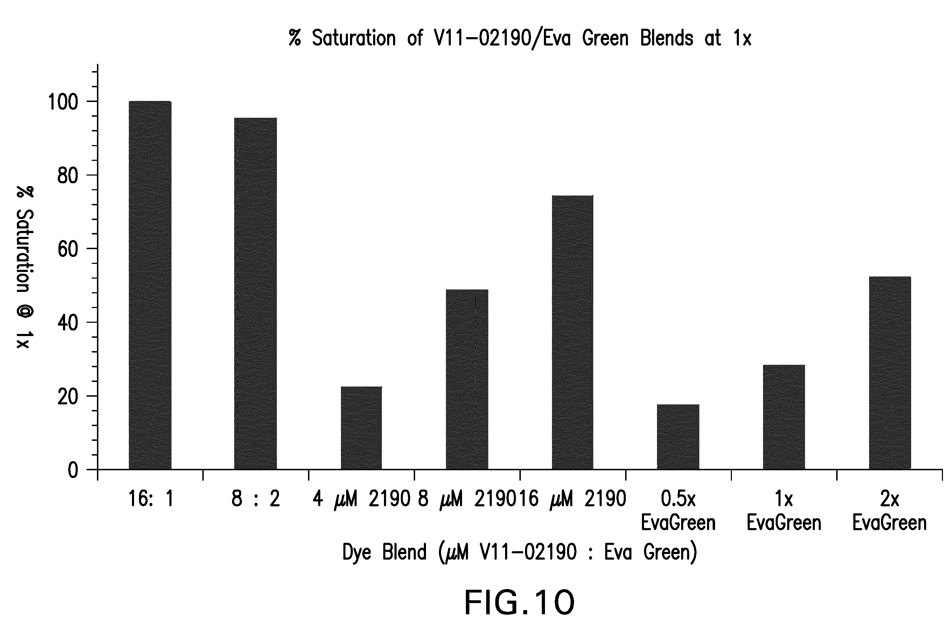

Dye blend 8 µM V11-02190 and 2X Eva Green®, and dye blend 16 µM V11-02190 and 1 X Eva Green®, showed increased saturation compared with either dye alone (V11-02190 control and Eva Green® control. Selected results from FIGS. 9A-9D were expressed in FIG. 10 as percent saturation at 1X. Percent saturation at 1X of dye blend V11-02190 and Eva Green® were equivalent to the sum of each controls (each dye alone). This indicated a complementary effect, unlike results with dye blend of V11-02190 and V11-03001, and indicated different modes of binding of V11-02190 and Eva Green® to dsDNA. Blending each of V11-02190 and Eva Green® improved the percent saturation at 1X of either dye alone.

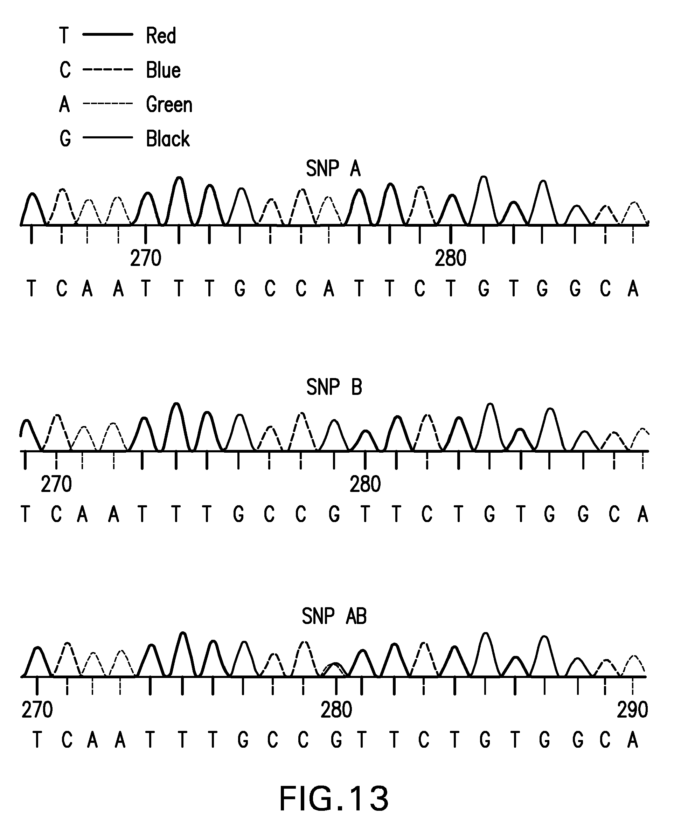

A dye blend of V11-02190 and a commercially available dye LC Green® (Idaho Technology) were evaluated at various concentrations, and the blend was compared with each dyes alone at various concentrations, in HRM analysis, with qRCR data of V11-02190 shown in FIG. 11, HRM data in FIG. 12C; 1 XLC Green qPCR data shown in FIG. 11 and HRM data in FIG. 12A. The performance of the dye blend in HRM was compared in qPCR reactions using a primer set that detects a 210 bp region GGATGGAAGA GATGACAAGG ACATAGGAGA TAAATATCAG GCCTATGGGC ACAAAAATCA CAAATGAACT TACACCATAA TTTATTATCT CATTGATAGT GGTATCAATG CAAGAAAGTT TCATGACTGG GTAAATGTCA CAAAAGAAGT GGTCTACCAC TGTGCCACAG AACGGCAAAT TGAACATGGC TGTCACATGG AGAACTGCCA (SEQ ID. NO: 3) the OR10J5 gene, with a forward primer sequence of 5' GGATGGAAGAGATGACAAGGACATAGG 3' (SEQ ID. NO: 4) and reverse primer sequence of 5' TGGCAGTTCTCCATGTGACAGC 3' (SEQ ID. NO: 5), the final concentration of each primer was 500 nM. Triplicate reactions from 3.5 ng, about 1000 copies, of three human genomic DNA samples, each known to contain one of each of 3 SNP genotypes A, B or AB as shown in FIG. 13 TCAATTTGCCATTCTGTGGCA (SEQ ID. NO: 6); TCAATTTGCCGTTCTGTGGCA (SEQ ID. NO: 7); as well as no template (water) control (NTC) reactions. PCR reactions were carried out using Dynamo Flash qPCR master mix (Thermo Scientific) on the Eco Real-Time PCR system (Illumina), with the following cycling protocol: 95°C for 7 minutes, followed by 50 cycles of 95°C for 10 seconds and 60°C for 15 seconds. For HRM analysis, ramp from 60°C to 95°C, rising by 0.1 °C each step. The effect of LC Green, V11-02190 alone and a combination of LC Green and V11-02190 (green dark dashed line) as the DNA-binding dye for real-time fluorescence monitoring, on PCR performance are shown in FIG. 11. Amplification plots of 1x LC Green alone (pink dark solid line), 20 µm V11-02190 alone (yellow dark dotted line), and a combination of 0.25x LC Green and 20 µm V11-02190 (green dark dashed line) as the DNA-binding dye for real-time fluorescence monitoring, where 1x indicates use at concentration from the manufacturer. Each template was amplified in triplicate under three human genomic DNA samples, each known to contain one of each of three SNP genotypes. In total, nine amplification plots representing each dye and dye blend. The delta R value (y-axis) and cycle number (x-axis) are shown.

FIGS. 12A-12C shows HRM analysis of the rs4656837 SNP (G/A) of OR10J5 gene. These data were obtained from Normalization Data and Difference Graphs, placing the pre-melting and post-melting parallel bars. Genomic DNA containing SNP A (blue dark dotted line), SNP B (green dark dashed line), and SNP AB (red or gray solid line) in a reaction mixture containing 1x LC green alone (FIG. 12A), 20 µm V11-02190 alone (FIG. 12B), and combination of 0.25x LC Green and 20 µm V11-02190 (FIG. 12C). The three genotypes were clearly distinguished. The G/G homozygote melted at the highest temperature (dark dashed line) while the A/A genotype melted at about 0.5°C lower (dark dotted line). The heterozygote is frequently easiest to distinguish with an altered melt profile (red or gray solid line). FIGS.12A-12C show difference graphs of the normalization data.

FIG. 13 shows a DNA sequencing result of SNP rs4656837. The sequencing was performed by using reverse primer of the PCR amplicon. The name SNP A indicates the SNP rs4656837 harbors A/A homozygote by reverse sequencing. The name SNP B indicates the SNP rs4656837 harbors G/G homozygote by reverse sequencing. The name SNP AB indicates the SNP rs4656837 harbors A/B heterozygote by reverse sequencing.

| | | | | | | | | | | | | | | | |
|---|---|---|---|---|---|---|---|---|---|---|---|---|---|---|---|
| **[Final] Eva Green ® (X)** | 0.2 5 | 0. 5 | 0.7 5 | 0 | 0 | 0 | 0.2 5 | 0. 5 | 0.7 5 | 0.2 5 | 0. 5 | 0.7 5 | 0.2 5 | 0. 5 | 0.7 5 |
| **[Final] LC Green (X)** | 0 | 0 | 0 | 0. 5 | 1 | 2 | 0.5 | 0. 5 | 0.5 | 1 | 1 | 1 | 2 | 2 | 2 |

A dye blend of EVAGREEN® and LCGREEN® (Idaho Technology) was evaluated at various concentrations. Each dye was added alone at each of the different concentrations as control reactions. Single dyes were included as controls at 0.25X to 0.75X and 0.5X to 2X optimum concentrations of each dye of EVAGREEN® and LCGREEN®, respectively. Reactions were cycled on Corbett Rotor Gene 6000 thermal cycler using a 72 well block with the following conditions: 95°C for 10 minutes, followed by 40 cycles of 95°C for 10 seconds, 60°C for 15 seconds during which fluorescence data was acquired, following which a melting curve was acquired at 70°C-85°C, rising by 0.1 °C each step. The performance of the dye blend in HRM was compared in qPCR reactions using primer set that detects a 108 bp region (ACTGGGTAAA TGTCACAAAA GAAGTGGTCT ACCACTGTGC CACAGAACGG CAAATTGAAC ATGGCTGTCA CATGGAGAAC TGCCATAGTC AGACCAATGC CAAAGGAC (SEQ ID. NO: 8) of the OR10J5 gene, with a forward primer sequence of 5' GTC CTT TGG CAT TGG TCT GAC TA 3' (SEQ ID. NO: 9); and reverse primer sequence of 5' ACT GGG TAA ATG TCA CAA AAG AAG TG 3' (SEQ ID. NO: 10); the final concentration of each primer was 500 nM. Triplicate reactions from 10 ng, about 2900 copies, of three human genomic DNA samples, each known to contain one of each of 3 SNP genotypes, GG, AG or AA, as well as negative template control reactions.

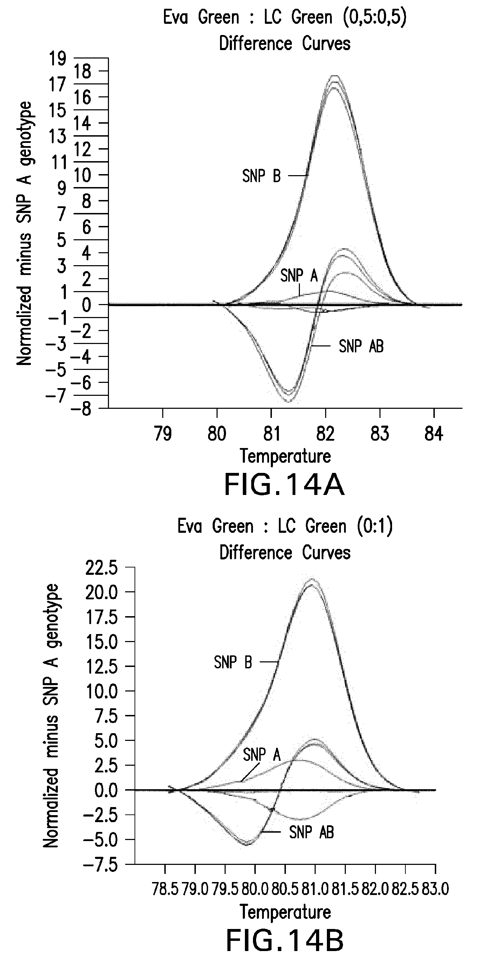

FIG. 14 shows high resolution melting (HRM) difference curves using dsDNA binding dyes separately and in combiantion. FIG. 14A shows HRM using a blend of EVAGREEN® (0.5X) and LCGREEN® (0.5X) (Eva Green: LC Green (0.5 : 0.5)). FIG. 14B shows HRM using LCGREEN® alone at 1X (14B) ((Eva Green: LC Green (0 : 1)). The results showed that using dye blend increased eficiency of HRM performance; the differences between the Tm of different genotypes were larger than when using LCGREEN® dye alone at recommended 1X concentration. FIGS. 14A-B shows high resolution melting (HRM) data using double stranded DNA (dsDNA) binding dyes separately and in combination.

Using HRM analysis, the dye blend that produced the combined highest RFU were 0.5X EVAGREEN® and 0.5X LCGREEN®.
The disclosed dye blends have properties that each dye alone does not provide.

As one example, dye blend V11-03001 and V11-02190 improved the fluorescence signal and, although to a lesser extent, improved Cq without increasing the concentration of either dye alone to a concentration that would be inhibitory, thus providing an improved dye composition for qPCR. Dye blend V11-03001 and V11-02190 provided a small improvement in dye saturation, thus providing an improved dye composition for HRM assays.

As one example, dye blend V11-02190 and EVAGREEN® improved the fluorescence signal compared with either dye alone, and improved Cq compared with EVAGREEN® alone. Dye blend V11-02190 and EVAGREEN® greatly improved dye saturation, thus providing an improved dye composition for HRM assays. Without being held to a single theory, it is believed that this effect may indicate that dyes having different structures in a dye blend have different modes of binding to dsDNA, and that the higher saturation results from an additive effect.

Mixing two different dsDNA binding dyes demonstrated improved properties compared with the individual dyes alone. The dye blends had higher fluorescence, and the same or lower Cq compared to each dye alone. The dye blends resulted in an additive effect on dsDNA saturation, which was achieved at dye concentrations that were not inhibitory to qPCR. The dye blends showed an additional effect beyond simply increasing the concentration of the individual dyes which, due to higher concentrations of either dye alone, exceeded the optimal concentration and inhibited DNA polymerase. The dye blends showed a synergistic effect by improving performance beyond the performance of each dye alone. This synergistic effect may reflect different binding modes on the dsDNA, as this effect was demonstrated to a greater extent when two structurally dissimilar dyes were in the dye blend, e.g., V11-02190 and EvaGreen.

As one example, dye blend V11-02190 and LCGREEN® improved the fluorescence signal and improved Cq compared to each dye alone. It provided an improved dye composition for HRM assay. This indicated that two structurally different dyes in a dye blend have different modes of binding to dsDNA, as the result of a dye blend shows a synergistic effect.

As one example, dye blend EVAGREEN® and LCGREEN® improved the dye saturation percentage, thus providing an improved dye composition for HRM assays. The dye blends showed a synergistic effect by improving performance beyond the HRM performance of each dye alone.

Without being bound to a single theory, the improved characteristics of the described dsDNA binding dye blends may increase the design space for dye concentration in a mix for HRM and qPCR. Using the blend of dsDNA binding dyes permits fluorescence maxima to be achieved at a lower concentration of each dye than using each individual dye alone, and at a concentration that is less inhibitory to DNA polymerase enzymes. The dye blends improved the fluorescence characteristics of qPCR and HRM, and also overcame the problems of qPCR inhibition associated with saturating concentrations of dsDNA binding dyes.

## Claims

1. A method for quantitative polymerase chain reaction (qPCR), the method comprising
combining a nucleic acid with a polymerase chain reaction mixture to form a sample, the polymerase chain reaction mixture comprising a thermostable polymerase, at least one primer oligonucleotide, and a first and a second double stranded DNA (dsDNA) binding dye, the first dsDNA binding dye is V11-02190 and the second dsDNA binding dye is selected from V11-03001, Eva Green@, or LC Green@,
performing the polymerase chain reaction on the sample to amplify the nucleic acid, to result in an amplified dsDNA , the polymerase chain reaction comprising subjecting the sample to a plurality of temperature cycles, each cycle further comprising exposing the sample to light at a wavelength absorbed by each of the dsDNA binding dyes and detecting a fluorescent emission intensity from the dsDNA binding dyes where the intensity is proportional to the quantity of amplified nucleic acid in the sample, where the dsDNA binding dyes have substantially similar excitation and emission spectra such that each of the two dsDNA binding dyes can be excited, and their resulting emissions detected, simultaneously.

2. The method of claim 1 further comprising subjecting the amplified dsDNA to a high resolution melting assay, the high resolution melting assay comprising
heating the amplified dsDNA to a melting temperature in the presence of the first and second dsDNA binding dyes, plotting the melting temperatures against fluorescence emitted from the dsDNA binding dyes to generate a melting curve, and identifying a genotype of the amplified dsDNA from the melting curve by melting temperature, melting curve slope, and/or melting curve shape.

3. The method of claim 1 or claim 2 wherein the excitation and emission spectra are substantially similar such that a single instrument configuration can excite and measure emission from the dyes in a single measurement.

4. The method of claim 3 wherein the single instrument configuration comprises a filter set which selects a range of excitation and emission wavelengths.

5. The method of any of claims 1 to 4 wherein the excitation is 488 nm ± 10 nm and the emission is 525 nm ± 10 nm.

6. The method of any of claims 1 to 4 wherein the excitation is 488 nm ± 20 nm and the emission is 525 nm ± 20 nm.

7. The method of any of claims 1 to 4 wherein the excitation is 488 nm ± 40 nm and the emission is 525 nm ± 40 nm.

8. The method of any of claims 1 to 7 further comprising the steps of:
exposing the polymerase chain reaction assay to light at a wavelength absorbed by each dsDNA binding dye; and
detecting a fluorescent emission intensity from the dsDNA binding dyes where the intensity is proportional to the quantity of the amplified target nucleic acid in the polymerase chain reaction assay;
wherein a target nucleic acid in a polymerase chain reaction assay is quantitated and/or detected.

9. The method of claim 8 where the target nucleic acid is a ribonucleic acid (RNA), and the method further comprises reverse transcribing the RNA into DNA prior to performing qPCR.

10. A method of analyzing a double-stranded deoxyribonucleic acid (dsDNA) in a high resolution melting assay, the method comprising
combining the dsDNA with a first and a second double stranded DNA (dsDNA) binding dye, the first dsDNA binding dye is V11-02190 and the second dsDNA binding dye is selected from V11-03001, Eva Green@, or LC Green@, to form a sample,
incrementally heating the sample while exposing the sample to light at a wavelength absorbed by each of the dsDNA binding dyes and detecting a fluorescent emission intensity from the dsDNA binding dyes, to produce melting temperatures of the dsDNA,
plotting the melting temperatures against fluorescence emitted from the dyes to generate a melting curve, and
analyzing the dsDNA from the melting curve by at least one of melting temperature, melting curve slope, or melting curve shape.

11. The method of claim 10 where the melting curve of the target dsDNA is compared with a melting curve of a reference dsDNA to determine a genotype of the target dsDNA.

12. A kit comprising a thermostable polymerase and a first and a second double stranded DNA (dsDNA) binding dye, the first dsDNA binding dye is V11-02190 and the second dsDNA binding dye is selected from V11-03001, Eva Green@, or LC Green@ and instructions for performing each of high resolution melting (HRM) and quantitative polymerase chain reaction (PCR) using the components of the kit.

13. The kit of claim 12 further comprising a reference nucleic acid encoding a gene selected from the group consisting of *CFTR, BRCA1, BRCA2, MFN2, HTT, SMPD1,* and *NOD2.*

## Patentansprüche

1. Verfahren zur quantitativen Polymerase-Kettenreaktion (qPCR), wobei das Verfahren umfasst:
Vereinigen einer Nucleinsäure mit einer Polymerase-Kettenreaktionsmischung zur Erzeugung einer Probe, wobei die Polymerase-Kettenreaktionsmischung eine thermostabile Polymerase, mindestens ein Primer-Oligonucleotid und einen ersten und einen zweiten doppelsträngige DNA (dsDNA)-bindenden Farbstoff umfasst, wobei der erste dsDNA-bindende Farbstoff V11-02190 ist und der zweite dsDNA-bindende Farbstoff ausgewählt ist aus V11-03001 EVA Green^{®} oder LC Green^{®},
Ausführen der Polymerase-Kettenreaktion auf der Probe zum Amplifizieren der Nucleinsäure, um eine amplifizierte dsDNA zu ergeben, wobei die Polymerase-Kettenreaktion das Unterwerfen der Probe einer Mehrzahl von Temperaturzyklen umfasst, wobei jeder Zyklus ferner das Exponieren der Probe an Licht bei einer Wellenlänge umfasst, die von jedem der beiden dsDNA-bindenden Farbstoffe absorbiert wird, und Detektieren der Intensität der Fluoreszenzemission von den dsDNA-bindenden Farbstoffen, wobei die Intensität proportional der Menge der amplifizierten Nucleinsäure in der Probe ist, wobei die dsDNA-bindenden Farbstoffe im Wesentlichen die gleichen Anregungsund Emissionsspektren haben, so dass jeder der zwei dsDNA-bindenden Farbstoffe simultan angeregt und deren Emissionen detektiert werden können.

2. Verfahren nach Anspruch 1, ferner umfassend, die amplifizierte dsDNA einem hochauflösenden Schmelz-Assay zu unterziehen, wobei das hochauflösende Schmelz-Assay umfasst:
Erhitzen der amplifizierten dsDNA bis zu einer Schmelztemperatur in Gegenwart der ersten und zweiten dsDNA-bindenden Farbstoffe, Auftragen der Schmelztemperaturen in Abhängigkeit von der Fluoreszenz, die von den dsDNA-bindenden Farbstoffen emittiert wird, um eine Schmelzkurve zu erzeugen, und identifizieren eines Genotyps der amplifizierten dsDNA aus der Schmelzkurve anhand der Schmelztemperatur, der Steigung der Schmelzkurve und/oder der Form der Schmelzkurve.

3. Verfahren nach Anspruch 1 oder 2, wobei die Anregungs- oder Emissionsspektren weitgehend ähnlich sind, so dass eine einzige Instrumentenkonfiguration die Emission von den Farbstoffen in einer einzigen Messung anregen und messen kann.

4. Verfahren nach Anspruch 3, wobei die einzige Instrumentenkonfiguration einen Filtersatz umfasst, der einen Bereich von Anregungs- und Emissionswellenlängen auswählt.

5. Verfahren nach Anspruch 1 bis 4, wobei die Anregung 488 nm ± 10 nm und die Emission 525 nm ± 10 nm betragen.

6. Verfahren nach Anspruch 1 bis 4, wobei die Anregung 488 nm ± 20 nm und die Emission 525 nm ± 20 nm betragen.

7. Verfahren nach Anspruch 1 bis 4, wobei die Anregung 488 nm ± 40 nm und die Emission 525 nm ± 40 nm betragen.

8. Verfahren nach einem der Ansprüche 1 bis 7, ferner umfassend die Schritte:
Exponieren des Polymerase-Kettenreaktionsassays an Licht bei einer Wellenlänge, die von jedem dsDNA-bindenden Farbstoff absorbiert wird; und
Detektieren der Intensität einer Fluoreszenzemission von den dsDNA-bindenden Farbstoffen, wobei die Intensität proportional der Menge der amplifizierten Ziel-Nucleinsäure in dem Polymerase-Kettenreaktionsassay ist;
wobei die Ziel-Nucleinsäure in einem Polymerase-Kettenreaktionsassay quantifiziert und/oder detektiert wird.

9. Verfahren nach Anspruch 8, wobei die Ziel-Nucleinsäure eine Ribonucleinsäure (RNA) ist und das Verfahren ferner das reverse Transkribieren der RNA in die DNA vor der Ausführung der qPCR umfasst.

10. Verfahren zum Analysieren einer doppelsträngigen Desoxyribonucleinsäure (dsDNA) in einem hochauflösenden Schmelz-Assay, wobei das Verfahren umfasst:
Vereinigen der dsDNA mit einem ersten und einem zweiten doppelsträngige DNA (dsDNA)-bindenden Farbstoff, wobei der erste dsDNA-bindende Farbstoff V11-02190 ist und der zweite dsDNA-bindende Farbstoff ausgewählt ist aus V11-03001 EVA Green^{®} oder LC Green^{®}, um eine Probe zu erzeugen,
schrittweises Erhitzen der Probe, während die Probe an Licht bei einer Wellenlänge exponiert wird, die von jedem der dsDNA-bindenden Farbstoffe absorbiert wird, und Detektieren der Intensität einer Fluoreszenzemission von den dsDNA-bindenden Farbstoffen, um Schmelztemperaturen der dsDNA zu erzeugen,
Auftragen der Schmelztemperaturen in Abhängigkeit von der Fluoreszenz, die von den Farbstoffen emittiert wird, um eine Schmelzkurve zu erzeugen, und
Analysieren der dsDNA aus der Schmelzkurve anhand mindestens einer der Schmelztemperaturen, der Steigung der Schmelzkurve oder der Form der Schmelzkurve.

11. Verfahren nach Anspruch 10, wobei die Schmelzkurve der Ziel-dsDNA mit der Schmelzkurve einer Referenz-dsDNA verglichen wird, um einen Genotyp der Ziel-dsDNA zu bestimmen.

12. Kit, umfassend eine thermostabile Polymerase und einen ersten und einen zweiten doppelsträngige DNA (dsDNA)-bindenden Farbstoff, wobei der erste dsDNA-bindende Farbstoff V11-02190 ist und der zweite dsDNA-bindende Farbstoff ausgewählt ist aus Vll-0300l EVA Green^{®} oder LC Green^{®}, und Anweisungen zur Ausführung jeder des hochauflösenden Schmelzens (HRM) und der Polymerase-Kettenreaktion (PCR) unter Verwendung der Komponenten des Kits.

13. Kit nach Anspruch 12, ferner umfassend eine Referenz-Nucleinsäure, die ein Gen codiert, das ausgewählt ist aus der Gruppe, bestehend aus CFTR, BRCA1, BRCA2, MFN2, SMPD1 und NOD2.

## Revendications

1. Procédé de réaction en chaîne par polymérase quantitative (PCRq), le procédé comprenant les opérations consistant à
combiner un acide nucléique avec un mélange de réaction en chaîne par polymérase pour former un échantillon, le mélange de réaction en chaîne par polymérase comprenant une polymérase thermostable, au moins un oligonucléotide d'amorce, et un premier et un second colorant de liaison d'ADN double brin (ADNdb), le premier colorant de liaison d'ADNdbest est V11-02190 et le second colorant de liaison d'ADNdb est choisi parmi V11-03001, Eva Green® ou LC Green®,
réaliser la réaction en chaîne par polymérase sur l'échantillon pour amplifier l'acide nucléique, pour donner un ADNdb amplifié, la réaction en chaîne par polymérase comprenant l'opération consistant à soumettre l'échantillon à une pluralité de cycles de température, chaque cycle comprenant en outre les opérations consistant à exposer l'échantillon à la lumière à une longueur d'onde absorbée par chacun des colorants de liaison d'ADNdb et à détecter une intensité d'émission de fluorescence provenant des colorants de liaison d'ADNdb, l'intensité étant proportionnelle à la quantité d'acide nucléique amplifié dans l'échantillon, les colorants de liaison d'ADNdb des spectres d'excitation et d'émission sensiblement similaires de telle sorte que chacun des deux colorants de liaison d'ADNdb peut être excité, et leurs émissions résultante peuvent être détectées, simultanément.

2. Procédé selon la revendication 1, comprenant en outre l'opération consistant à soumettre de l'ADNdb amplifié à un dosage par fusion haute résolution, le dosage par fusion haute résolution comprenant les opérations consistant à
chauffer l'ADNdb amplifié à une température de fusion en présence des premier et second colorants de liaison d'ADNdb, tracer les courbes de températures de fusion en fonction de la fluorescence émise par les colorants de liaison d'ADNdb pour générer une courbe de fusion, et identifier un génotype de l'ADNdb amplifié à partir de la courbe de fusion selon la température de fusion, la pente de la courbe de fusion, et/ou de la forme de la courbe de fusion.

3. Procédé selon la revendication 1 ou la revendication 2, dans lequel les spectres d'excitation et d'émission sont sensiblement similaires de sorte qu'une seule configuration d'instrument peut exciter et mesurer l'émission provenant des colorants en une seule mesure.

4. Procédé selon la revendication 3, dans lequel la seule configuration d'instrument comprend un ensemble de filtre qui sélectionne une gamme de longueurs d'ondes d'excitation et d'émission.

5. Procédé selon l'une quelconque des revendications 1 à 4, dans lequel l'excitation est de 488 nm ± 10 nm et l'émission est de 525 nm ± 10 nm.

6. Procédé selon l'une quelconque des revendications 1 à 4, dans lequel l'excitation est de 488 nm ± 20 nm et l'émission est de 525 nm ± 20 nm.

7. Procédé selon l'une quelconque des revendications 1 à 4, dans lequel l'excitation est de 488 nm ± 40 nm et l'émission est de 525 nm ± 40 nm.

8. Procédé selon l'une quelconque des revendications 1 à 7, comprenant en outre les étapes consistant à :
exposer le dosage par réaction en chaîne par polymérase à la lumière à une longueur d'onde absorbée par chaque colorant de liaison d'ADNdb ; et
détecter une intensité d'émission de fluorescence provenant des colorants de liaison d'ADNdb, l'intensité étant proportionnelle à la quantité d'acide nucléique cible amplifié dans le dosage par réaction en chaîne par polymérase ;
un acide nucléique cible dans un dosage par réaction en chaîne par polymérase étant quantifié et/ou détecté.

9. Procédé selon la revendication 8 dans lequel l'acide nucléique cible est un acide ribonucléique (ARN), et le procédé comprend en outre la transcription inverse de l'ARN en ADN avant d'effectuer la PCRq.

10. Procédé d'analyse d'un acide désoxyribonucléique double brin (ADNdb) dans un dosage par fusion haute résolution, le procédé comprenant les étapes consistant à
combiner l'ADNdb avec un premier et un second colorant de liaison d'ADN double brin (ADNdb), le premier colorant de liaison d'ADNdb étant V11-02.190 et le second colorant de liaison d'ADNdb étant choisi parmi V11-03.001, Eva Green®, ou LC Green®, pour former un échantillon,
chauffer de façon incrémentielle l'échantillon tout en exposant l'échantillon à une lumière de longueur d'onde absorbée par chacun des colorants de liaison d'ADNdb et détectant une intensité d'émission de fluorescence des colorants de liaison d'ADNdb, pour produire des températures de fusion de l'ADNdb,
tracer les courbes de températures de fusion en fonction de la fluorescence émise par les colorants pour générer une courbe de fusion, et
analyser l'ADNdb à partir de la courbe de fusion selon au moins une parmi la température de fusion, la pente de la courbe de fusion, ou la forme de la courbe de fusion.

11. Procédé selon la revendication 10, dans lequel la courbe de fusion de l'ADNdb cible est comparée à une courbe de fusion d'un ADNdb de référence pour déterminer un génotype de l'ADNdb cible.

12. Kit comprenant une polymérase thermostable et un premier et un second colorant de liaison d'ADN double brin (ADNdb), le premier colorant de liaison d'ADNdb étant V11-02190 et le second colorant de liaison d'ADNdb étant choisi parmi V11-03001, Eva Green®, ou LC Greer® et des instructions pour réaliser une réaction par fusion haute résolution (HRM) et une réaction en chaîne par polymérase (PCR) quantitative à l'aide des composants du kit.

13. Kit selon la revendication 12, comprenant en outre un acide nucléique de référence codant un gène choisi dans le groupe comportant *CFTR, BRCA1*, *BRCA2, MFN2, HTT, SMPD1,* et *NOD2.*
